(19) Europäisches Patentamt / European Patent Office / Office européen des brevets

(11) **EP 2 056 771 B1**

(12) **EUROPEAN PATENT SPECIFICATION**

(45) Date of publication and mention
of the grant of the patent:
**28.07.2010 Bulletin 2010/30**

(51) Int Cl.:
***A61F 13/42*** *(2006.01)*

(21) Application number: **07826028.8**

(86) International application number:
**PCT/IB2007/053236**

(22) Date of filing: **14.08.2007**

(87) International publication number:
**WO 2008/026123 (06.03.2008 Gazette 2008/10)**

(54) **CONDUCTION THROUGH A FLEXIBLE SUBSTRATE IN AN ARTICLE**

LEITUNG DURCH EIN FLEXIBLES SUBSTRAT BEI EINEM ARTIKEL

CONDUCTION DANS UN SUBSTRAT SOUPLE D'UN ARTICLE

(84) Designated Contracting States:
**DE GB**

(30) Priority: **31.08.2006 US 514542**

(43) Date of publication of application:
**13.05.2009 Bulletin 2009/20**

(73) Proprietor: **KIMBERLY-CLARK WORLDWIDE, INC.
Neenah, WI 54956 (US)**

(72) Inventors:
• **TIPPEY, Darold, Dean
Canton, 61520 Illinois (US)**

• **ALES, III, Thomas, Michael
Neenah, Wisconsin 54956 (US)**

(74) Representative: **Zimmermann & Partner
Postfach 330 920
80069 München (DE)**

(56) References cited:
**EP-A- 1 047 033      US-A- 5 646 369
US-B1- 6 200 250**

**Description**

**BACKGROUND**

Electrical circuits have been printed or otherwise applied to flexible substrates, such as paper, woven fabrics, nonwoven fabrics and polymer films. The electrical circuits have incorporated electrically conductive inks applied with conventional ink-printing techniques, and various products, such as badges, labels and tags, have incorporated the printed circuits. In particular arrangements, the printed circuits have been employed in hygienic products, such as drapes, gowns, garments, personal care absorbent products; and the like. In other arrangements, electrical/electronic circuitry has been employed to provide sensors located in selected personal care products, such as wetness sensors located in disposable infant diapers.

Conventional ink-printed circuit configurations, however, have included a printing of conductive ink on one side of a single layer of a polymer film or other flexible substrate composed of electrically-insulating material. Ink-printed circuits positioned on a single side of an insulating substrate can present problems when attempting to connect the printed circuit to a cooperating sensor or other external electrical monitoring device that is positioned on an opposite side of the insulating substrate. Interconnecting, conductive pathways formed through the thickness of the insulating substrate have excessively compromised the desired properties of the substrate. For example, where the substrate is desired to be substantially liquid-impermeable, the formation of the conductive pathway through the thickness of the insulating substrate has excessively degraded the desired level of liquid-impermeability. As a result, when the liquid-impermeable substrate and cooperating monitoring device have been employed on a garment, it has been necessary to position the interconnecting, conductive pathway at a garment location that can cause excessive irritation to a wearer of the garment. In addition, it has been difficult to form a strong and reliable electrical and mechanical interconnection that can sufficiently resist failure due to mechanical stress-fatigue generated by movements of the substrate material during the wearer's activities.

**SUMMARY**

In response to the needs discussed above, an article of the present invention includes providing a first electrically conductive circuit-path, and separately providing at least a second electrically conductive circuit-path. A portion of the first circuit-path is positioned proximally adjacent a portion of the second circuit-path at a first predetermined hole location. A first, electrically-insulating barrier layer is interposed between the first circuit-path and second circuit-path at the first hole location, and the first circuit-path is conductively connected to the second circuit-path at the first hole location by filling the hole with a conductive filler to form a continuous electrical circuit. The connection is configured to provide an electrically conductive path between the first circuit-path and the second circuit-path at the first hole location. The conductive filler is configured such that the first circuit-path is conductively connected to the second circuit-path at the first predetermined hole location to form an interconnecting conductive filler-path between the first circuit-path and the second circuit-path. In some aspects, the first electrically conductive circuit-path is operatively connected to a sensor mechanism which provides sensor data. In further aspects, the second electrically conductive circuit-path can be operatively connected to an electronic processor mechanism which receives the sensor data and provides selected signal data.

By incorporating its various aspects and features, the invention can provide a desired, electrically-conductive interconnecting pathway between electrically-conductive circuit-paths that are positioned on opposite sides of a substrate which is configured to be an electrically-insulating layer. The interconnecting conductive pathway desirably penetrates and extends through the thickness dimension of the substrate, and the formation of the interconnecting conductive pathway can be configured to operatively maintain desired properties of the substrate. For example, the formation of the interconnecting conductive pathway can be configured to operatively maintain a desired liquid-impermeable barrier property of the substrate. As a result, the interconnecting conductive pathway can be positioned at a greater range of locations and can help provide greater versatility. For example, when the conductive pathway interconnects a circuit-path that is positioned on one side of the insulating substrate to a cooperating sensor or other external electrical monitoring device that is positioned on an opposite side of the substrate, the conductive pathway can have a location that allows a cooperative positioning of the monitoring device at a location that provides improved comfort to the wearer.

Numerous other features and advantages of the present invention will appear from the following description. In the description, reference is made to exemplary embodiments of the invention. Such embodiments do not represent the full scope of the invention. Reference should therefore be made to the claims herein for interpreting the full scope of the invention.

**BRIEF DESCRIPTION OF THE DRAWINGS**

The foregoing and other features, aspects and advantages of the present invention will become better understood with regard to the following description, appended claims and accompanying drawings where:

FIG 1 is a perspective view of one embodiment of an absorbent article that may be made in accordance with the present invention;

FIG 2 is a plan view of the absorbent article shown in FIG 1 with the article in an unfastened, unfolded and laid flat condition showing the surface of the article that faces the wearer when worn and with portions cut away to show underlying features;

FIG 3A is a cross-section side view of an absorbent bandage of the present invention;

FIG 3B is a top perspective view of an absorbent bandage of the present invention;

FIG 4 is a top perspective view of an absorbent bed or furniture liner of the present invention;

FIG 5 is a perspective view of an absorbent sweatband of the present invention;

FIG 6 is a perspective view of a glove of the present invention.

FIG 7 shows a top plan view of a representative, barrier layer substrate in which a first circuit-path is located on a first; major facing-surface of the substrate is connected to a second circuit-path located on an opposite, second, major facing-surface of the substrate with an electrically-conductive filler-path conductively connecting the first and second circuit-paths.

FIG 7A shows a representative cross-sectional view of a substrate and associated circuit-paths taken along section 1A - 1A of FIG 7.

FIG 8 shows a top plan view of a representative arrangement wherein a first circuit-path is located on a first substrate; a second circuit-path is located on a second substrate: a third circuit-path is located on a third substrate; the first circuit-path is connected to the second circuit-path along a first electrically conductive filler-path through the thickness of the second substrate; and the second circuit-path is connected to the third circuit-path along a second electricallyconductive filler-path through the thickness of the third substrate.

FIG 9 shows a representative, schematic arrangement for determining the resistance of an electricallyconductive filler-path.

FIG 10 shows a partially cut-away, perspective view of a representative article having a sensor or other external electrical monitoring device that is positioned on an inward side surface of the insulating substrate and interconnects through a hole in the substrate that has been filled with a conductive filler to a circuit-path and cooperating processor that is positioned on an opposite, outward side of the substrate.

FIG 11 shows a perspective, partial view of another representative arrangement of a first-circuit path that is positioned over a hole that is filled with a conductive filler through the substrate to a second circuit-path and cooperating processor that is positioned on an opposite, outward-side surface of the substrate.

## DEFINITIONS

It should be noted that, when employed in the present disclosure, the terms "comprises", "comprising" and other derivatives from the root term "comprise" are intended to be open-ended terms that specify the presence of any stated features, elements, integers, steps, or components, and are not intended to preclude the presence or addition of one or more other features, elements, integers, steps, components, or groups thereof.

The term "conductive filler" refers to a liquid or solid substance capable of conducting an electrical charge that is applied to at least one hole in a substrate of the present invention such that a conductive connection is formed between at least a first circuit-path positioned on one side of the substrate, and a second circuit-path positioned proximally adjacent to the first circuit-path but on the opposite side of the substrate. When utilized in a liquid impervious substrate, the conductive filler substantially maintains the liquid impervious properties of the substrate.

The term "continuous electrical circuit" refers to a circuit that is not interrupted at the point where the conduction occurs through the substrate.

The term "disposable" is used herein to describe articles that are not intended to be laundered or otherwise restored or reused after a single use. Examples of such disposable articles include, but are not limited to, personal care articles, health/medical articles, household/industrial articles, and sports/construction articles.

The terms "fluid impermeable," "liquid impermeable," "fluid impervious" and "liquid impervious" mean that fluid such as water or bodily fluids will not pass substantially through the layer or laminate under ordinary use conditions in a direction generally perpendicular to the plane of the layer or laminate at the point of fluid contact.

The term "health/medical articles" includes a variety of professional and consumer health-care products including, but not limited to, products for applying hot or cold therapy, medical gowns (i.e., protective and/or surgical gowns), surgical drapes, caps, gloves, face masks, bandages, wound dressings, wipes, covers, containers, filters, disposable garments and bed pads, medical absorbent garments, underpads, and the like.

The term "household/industrial absorbent articles" includes construction and packaging supplies, products for cleaning and disinfecting, wipes, covers, gloves, filters, towels, disposable cutting sheets, bath tissue, facial tissue, nonwoven roll goods, home-comfort products including pillows, pads, mats, cushions, masks and body care products such as products used to cleanse or treat the skin, laboratory coats, cover-alls, trash bags, stain removers, topical compositions, pet care absorbent liners, laundry soil/ink absorbers, detergent agglomerators, lipophilic fluid separators, and the like.

The term "MD" or "machine direction" refers to the orientation of the absorbent web that is parallel to the running direction of the forming fabric and generally within the plane formed by the forming surface. The term "CD" or "cross-machine direction" or "cross deckle" refer to the direction perpendicular to the MD and generally within the plane formed by the forming surface. Both MD and CD generally define a plane that is parallel to the forming surface. The term "ZD "or "Z-direction" refers to the orientation that is perpendicular to the plane formed by the MD and CD.

As used herein, the phrase "meltblown fibers" refers to fibers formed by extruding a molten thermoplastic material through a plurality of fine, usually circular, die capillaries as molten threads or filaments into a high velocity, usually heated, gas (e.g., air) stream which attenuates the filaments of molten thermoplastic material to reduce their diameter. Thereafter, the meltblown fibers are carried by the high velocity gas stream and are deposited on a collecting surface to form a web of randomly disbursed meltblown fibers. "Coform" as used herein is intended to describe a blend of meltblown fibers and cellulose fibers that is formed by air forming a meltblown polymer material while simultaneously blowing air-suspended cellulose fibers into the stream of meltblown fibers. The meltblown fibers containing wood fibers are collected on a forming surface, such as provided by a foraminous belt. The forming surface may include a gas-pervious material, such as spunbonded fabric material, that has been placed onto the forming surface.

As used herein, the term "nonwoven" refers to a fabric web that has a structure of individual fibers or filaments which are interlaid, but not in an identifiable repeating manner.

By the terms "particle," "particles," "particulate," "particulates" and the like, it is meant that the material is generally in the form of discrete units. The units can comprise granules, powders, spheres, pulverized materials or the like, as well as combinations thereof. The particles can have any desired shape such as, for example, cubic, rod-like, polyhedral, spherical or semi-spherical, rounded or semi-rounded, angular, irregular, etc. Shapes having a large greatest dimension/smallest dimension ratio, like needles, flakes and fibers, are also contemplated for inclusion herein. The terms "particle" or "particulate" may also include an agglomeration comprising more than one individual particle, particulate or the like. Additionally, a particle, particulate or any desired agglomeration thereof may be composed of more than one type of material.

The term "personal care article" includes, but is not limited to, absorbent articles such as diapers, diaper pants, baby wipes, training pants, absorbent underpants, child care pants, swimwear, and other disposable garments; feminine care products including sanitary napkins, wipes; menstrual pads, menstrual pants, panty liners, panty shields, interlabials, tampons, and tampon applicators; adult-care products including wipes, pads such as breast pads, containers, incontinence products, and urinary shields; clothing components; bibs; athletic and recreation products; and the like.

The term "sports/construction articles" includes headbands, wrist bands and other aids for absorption of perspiration, absorptive windings for grips and handles of sports equipment, and towels or absorbent wipes for cleaning and drying off equipment during use.

As used herein, the terms "spunbond" or "spunbonded fiber" refer to fibers which are formed by extruding filaments of molten thermoplastic material from a plurality of fine, usually circular, capillaries of a spinneret, and then rapidly reducing the diameter of the extruded filaments.

As used herein, the term "substrate" may include one or more layers.

These terms may be defined with additional language in the remaining portions of the specification.

## DETAILED DESCRIPTION

Disposable articles such as, for example, many personal care products, can include at least one of a liquid pervious topsheet, a substantially liquid impervious backsheet which may be joined to the topsheet, and an absorbent core positioned and held between the topsheet and the backsheet. The topsheet can be operatively permeable to the liquids that are intended to be held or stored by the article, and the backsheet may be substantially impermeable or otherwise operatively impermeable to the intended liquids. The disposable article may also include other components, such as liquid wicking layers, liquid distribution layers, barrier layers, and the like, as well as combinations thereof. Disposable articles and the components thereof, can operate to provide a body-facing surface and a garment-facing surface. As used herein, "body-facing surface" means that surface of the article or component which is intended to be disposed toward or placed adjacent to the body of the wearer during ordinary use, while the "outward surface" or "outward-facing surface" is on the opposite side, and is intended to be disposed to face away from the wearer's body during ordinary use. The outward surface may be arranged to face toward or placed adjacent to the wearer's undergarments when the article is worn.

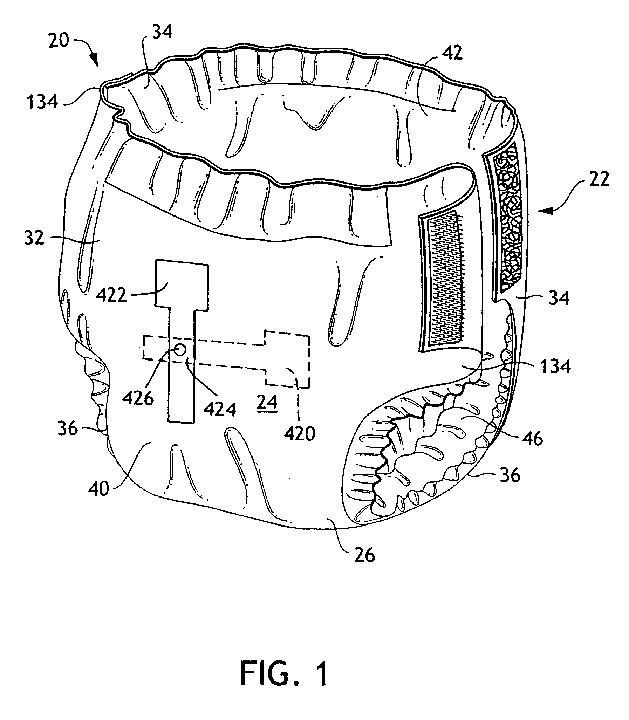

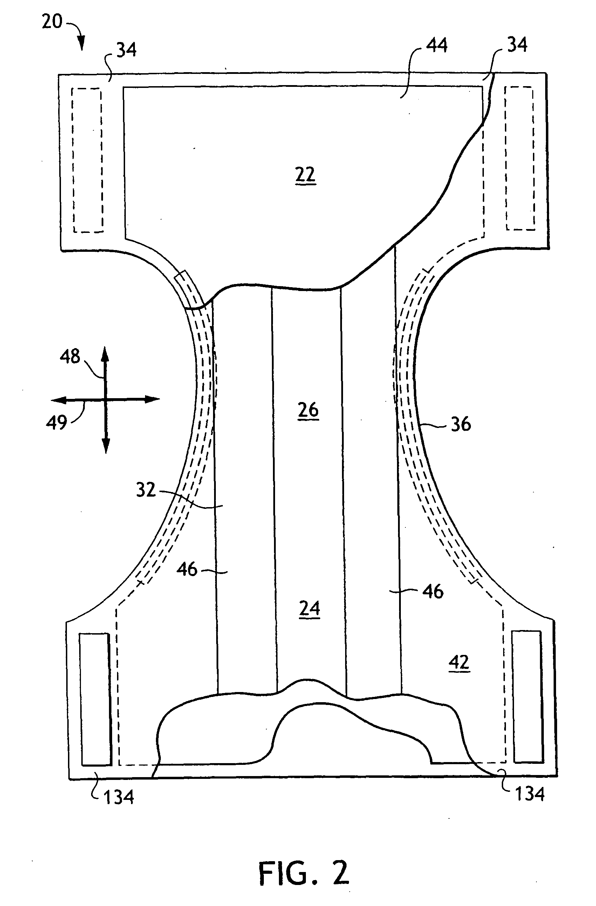

To gain a better understanding of the present invention, attention is first directed to FIG 1 which illustrates a training pant in a partially fastened condition, and to FIG 2 illustrates a training pant in an opened and unfolded state. By way of a non-limiting example, such a training pant could, in some aspects, comprise a wetness alarm that could be placed anywhere on the article when utilizing the invention of the present disclosure. It is understood that the present invention is suitable for use with various other disposable articles, including but not limited to other personal care articles, health/medical articles, household/industrial articles, sports accessory articles and the like, without departing from the scope of the present invention.

Referring to FIG 1 and FIG 2, the training pant defines a longitudinal direction 48 that extends from the front of the training pant when worn to the back of the training pant. Perpendicular to the longitudinal direction 48 is a lateral direction 49.

The training pant defines a front region 22, a back region 24, and a crotch region 26 extending longitudinally between and interconnecting the front and back regions. The pant also defines an inner surface adapted in use (e.g., positioned relative to the other components of the pant) to be disposed toward the wearer, and an outer surface opposite the inner surface. The training pant has a pair of laterally opposite side edges and a pair of longitudinally opposite waist edges.

The illustrated pant 20 may include a chassis 32, a pair of laterally opposite front side panels 34 extending laterally outward at the front region 22 and a pair of laterally opposite back side panels 134 extending laterally outward at the back region 24.

The chassis 32 includes substrates including a backsheet 40, and a topsheet 42 that may be joined to the backsheet 40 in a superimposed relation therewith such as by adhesives, ultrasonic bonds, thermal bonds or other conventional techniques. The chassis 32 further includes an absorbent core 44 such as shown in FIG 2 disposed between the backsheet 40 and the topsheet 42 for absorbing fluid body exudates exuded by the wearer, and may further include a pair of containment flaps 46 secured to the topsheet 42 or the absorbent core 44 for inhibiting the lateral flow of body exudates. The article can have a first circuit-path 420 disposed along a bodyside surface of at least one substrate, and at least a second circuit-path 422 that is proximally adjacent a portion of the first disposed along an outward, garment-side. The circuit-paths are conductively connected by a hole 424 that has been filled with a conductive filler 426.

The backsheet 40, the topsheet 42 and the absorbent core 44 may be made from many different materials known to those skilled in the art. Any of the three layers, for instance, may be extensible and/or elastically extensible. Further, the properties of each layer may vary in order to control the overall properties of the product.

The backsheet 40, for instance, may be breathable and/or may be fluid impermeable. The backsheet 40 may be constructed of a single layer, multiple layers, laminates, spunbond fabrics, films, meltblown fabrics, elastic netting, microporous webs or bonded-carded-webs. The backsheet 40, for instance, can be a single layer of a fluid impermeable material, or alternatively can be a multi-layered laminate structure in which at least one of the layers is fluid impermeable.

The backsheet 40 can be biaxially extensible and optionally biaxially elastic. Elastic non-woven laminate webs that can be used as the backsheet 40 include a non-woven material joined to one or more gatherable non-woven webs or films. Stretch Bonded Laminates (SBL) and Neck Bonded Laminates (NBL) are examples of elastomeric composites.

Examples of suitable nonwoven materials are spunbond-meltblown fabrics, spunbond-meltblown-spunbond fabrics, spunbond fabrics, or laminates of such fabrics with films, or other nonwoven webs. Elastomeric materials may include cast or blown films, meltblown fabrics or spunbond fabrics composed of polyethylene, polypropylene, or polyolefin elastomers, as well as combinations thereof. The elastomeric materials may include PEBAX elastomer (available from AtoFina Chemicals, Inc., a business having offices located in Philadelphia, Pennsylvania, U.S.A.), HYTREL elastomeric polyester (available from Invista, a business having offices located in Wichita, Kansas, U.S.A.), KRATON elastomer (available from Kraton Polymers, a business having offices located in Houston, Texas, U.S.A.), or strands of LYCRA elastomer (available from Invista), or the like, as well as combinations thereof. The backsheet 40 may include materials that have elastomeric properties through a mechanical process, printing process, heating process or chemical treatment. For example, such materials may be apertured, creped, neck-stretched, heat activated, embossed, and micro-strained, and may be in the form of films, webs, and laminates.

One example of a suitable material for a biaxially stretchable backsheet 40 is a breathable elastic film/nonwoven laminate, such as described in U.S. Patent 5,883,028, to Morman et al., Examples of materials having two-way stretchability and retractability are disclosed in U.S. Patents 5,116,662 to Morman and 5,114,781 to Morman, These two patents describe composite elastic materials capable of stretching in at least two directions. The materials have at least one elastic sheet and at least one necked material, or reversibly necked material, joined to the elastic sheet at least at three locations arranged in a nonlinear configuration, so that the necked, or reversibly necked, web is gathered between at least two of those locations.

The topsheet 42 is suitably compliant, soft-feeling and non-irritating to the wearer's skin. The topsheet 42 is also sufficiently liquid permeable to permit liquid body exudates to readily penetrate through its thickness to the absorbent core 44. A suitable topsheet 42 may be manufactured from a wide selection of web materials, such as porous foams, reticulated foams, apertured plastic films, woven and non-woven webs, or a combination of any such materials. For example, the topsheet 42 may include a meltblown web, a spunbonded web, or a bonded-carded-web composed of natural fibers, synthetic fibers or combinations thereof. The topsheet 42 may be composed of a substantially hydrophobic material, and the hydrophobic material may optionally be treated with a surfactant or otherwise processed to impart a desired level of wettability and hydrophilicity.

In some aspects, the topsheet 42 may also be extensible and/or elastically extensible. Suitable elastomeric materials for construction of the topsheet 42 can include elastic strands, LYCRA elastics, cast or blown elastic films, nonwoven elastic webs, meltblown or spunbond elastomeric fibrous webs, as well as combinations thereof. Examples of suitable elastomeric materials include KRATON elastomers, HYTREL elastomers, ESTANE elastomeric polyurethanes (available from Noveon, a business having offices located in Cleveland, Ohio, U.S.A.), or PEBAX elastomers. The topsheet 42 can also be made from extensible materials such as those described in U.S. Patent 6,552,245 to Roessler et al. The topsheet 42 can also be made from biaxially stretchable materials as described in U.S. Patent 6,641,134 filed to Vukos et al.

In some aspects, one or more regions of the invention may be designed to have a function similar to the topsheet, which allows for the elimination of a separate topsheet layer.

The article 20 can further comprise an absorbent body structure, and the absorbent body can include an absorbent core 44 component. The absorbent core 44 may have any of a number of shapes. For example, it may have a 2-dimensional or 3-dimensional configuration, and may be rectangular shaped, triangular shaped, oval shaped, racetrack shaped. I-shaped, generally hourglass shaped, T-shaped and the like. It is often suitable for the absorbent core 44 to be narrower in the crotch portion 26 than in the rear 24 or front 22 portion(s). The absorbent core 44 can be attached in an absorbent article, such as to the backsheet 40 and/or the topsheet 42 for example, by bonding means known in the art, such as ultrasonic, pressure, adhesive, aperturing, heat, sewing thread or strand, autogenous or self-adhering, hook-and-loop, or any combination thereof.

In some aspects, the absorbent core 44 can have a significant amount of stretchability. For example, the absorbent core 44 can comprise a matrix of fibers which includes an operative amount of elastomeric polymer fibers. Other methods known in the art can include attaching superabsorbent materials to a stretchable film, utilizing a nonwoven substrate having cuts or slits in its structure, and the like.

The absorbent core 44 can be formed using methods known in the art. While not being limited to the specific method of manufacture, the absorbent core can utilize a meltblown process and can further be formed on a coform line. Exemplary meltblown processes are described in various patents and publications, including NRL Report 4364, "Manufacture of Super-Fine Organic Fibers" by V. A. Wendt, E. L. Boone and C. D. Fluharty; NRL Report 5265, "An Improved Device For the Formation of Super-Fine Thermoplastic Fibers" by K. D. Lawrence, R. T. Lukas and J. A. Young; and U.S. Patent Nos. 3,849,241 to Butin et al. and 5,350,624 to Georger et al.

To form "coform" materials, additional components are mixed with the meltblown fibers as the fibers are deposited onto a forming surface. For example, the superabsorbent materials and fluff, such as wood pulp fibers, may be injected into the meltblown fiber stream so as to be entrapped and/or bonded to the meltblown fibers. Exemplary coform processes are described in U.S. Patent Nos. 4,100,324 to Anderson et al.; 4,587,154 to Hotchkiss et al.; 4,604,313 to McFarland et al.; 4,655,757 to McFarland et al.; 4,724,114 to McFarland et al.; 4,100,324 to Anderson et al.; and U.K. Patent GB 2,151,272 to Minto et al., Absorbent, elastomeric meltblown webs containing high amounts of superabsorbent are described in U.S. Patent No. 6,362,389 to D. J. McDowall, and absorbent, elastomeric meltblown webs containing high amounts of superabsorbent and low superabsorbent shakeout values are described in pending U.S. Patent Application 10/883174 to X. Zhang et al.,

The absorbent core 44 also includes absorbent material, such as superabsorbent material and/or fluff. Additionally, the superabsorbent material can be operatively contained within a matrix of fibers, such as polymeric fibers. Accordingly, the absorbent core 44 can comprise a quantity of superabsorbent material and/or fluff contained within a matrix of fibers. In some aspects, the amount of superabsorbent material in the absorbent core 44 can be at least about 10% by weight of the core, such as at least about 30%, or at least about 60% by weight or at least about 90%, or between about 10% and about 99% by weight of the core, or between about 30% to about 90% by weight of the core to provide improved benefits. Optionally, the amount of superabsorbent material can be at least about 95% by weight of the core.

In other aspects, the absorbent core 44 can comprise about 35% or less by weight fluff, such as about 20% or less, or 10% or less by weight fluff.

It should be understood that the present invention is not restricted to use with superabsorbent materials and/or fluff. In some aspects, the absorbent core 44 may additionally or alternatively include materials such as surfactants, ion exchange resin particles, moisturizers, emollients, perfumes, natural fibers, synthetic fibers, fluid modifiers, odor control additives, and combinations thereof. Alternatively, the absorbent core 44 can include a foam.

In order to function well, the absorbent core 44 can have certain desired properties to provide improved performance as well as greater comfort and confidence among the user. For instance, the absorbent core 44 can have corresponding configurations of absorbent capacities, densities, basis weights and/or sizes which are selectively constructed and arranged to provide desired combinations of absorbency properties such as liquid intake rate, absorbent capacity, liquid distribution or fit properties such as shape maintenance and aesthetics. Likewise, the components can have desired wet to dry strength ratios, mean flow pore sizes, permeabilities and elongation values.

The polymer fibers of the absorbent core 44 can include an amount of a surfactant. The surfactant can be combined with the polymer fibers of the absorbent core in any operative manner. Various techniques for combining the surfactant are conventional and well known to persons skilled in the art. For example, the surfactant may be compounded with the polymer employed to form a meltblown fiber structure. In a particular feature, the surfactant may be configured to operatively migrate or segregate to the outer surface of the fibers upon the cooling of the fibers. Alternatively, the surfactant may be applied to or otherwise combined with the polymer fibers after the fibers have been formed.

The polymer fibers can include an operative amount of surfactant, based on the total weight of the fibers and surfactant. In some aspects, the polymer fibers can include at least a minimum of about 0.1 % by weight surfactant, as determined by water extraction. The amount of surfactant can alternatively be at least about 0.15% by weight, and can optionally be at least about 0.2% by weight to provide desired benefits. In other aspects, the amount of surfactant can be generally not more than a maximum of about 2% by weight, such as not more than about 1% by weight, or not more than about 0.5% by weight to provide improved performance.

The absorbent core 44 can optionally include fluff, such as cellulosic fibers. Such cellulosic fibers may include, but are not limited to, chemical wood pulps such as sulfite and sulfate (sometimes called Kraft) pulps, as well as mechanical pulps such as ground wood, thermomechanical pulp and chemithermomechanical pulp. More particularly, the pulp fibers may include cotton, other typical wood pulps, cellulose acetate, debonded chemical wood pulp, and combinations thereof. Pulps derived from both deciduous and coniferous trees can be used. Additionally, the cellulosic fibers may include such hydrophilic materials as natural plant fibers, milkweed floss, cotton fibers, microcrystalline cellulose, microfibrillated cellulose, or any of these materials in combination with wood pulp fibers. Suitable cellulosic fluff fibers can include, for example, NB480 (available from Weyerhaeuser Co.); NB416, a bleached southern softwood Kraft pulp (available from Weyerhaeuser Co.); CR 54, a bleached southern softwood Kraft pulp (available from Bowater Inc., a business having offices located in Greenville, South Carolina U.S.A.).; SULPHATATE HJ, a chemically modified hardwood pulp (available from Rayonier Inc., a business having offices located in Jesup, Georgia U.S.A.); NF 405, a chemically treated bleached southern softwood Kraft pulp (available from Weyerhaeuser Co., a business having offices located in Federal Way, Washington U.S.A.); and CR 1654, a mixed bleached southern softwood and hardwood Kraft pulp (available from Bowater Inc.)

As referenced above, the absorbent core 44 also includes a desired amount of superabsorbent material. Superabsorbent materials typically are polymers of unsaturated carboxylic acids or derivatives thereof. These polymers are rendered water insoluble, but water swellable, by crosslinking the polymer with a di- or poly-functional internal crosslinking agent. These internally crosslinked polymers are at least partially neutralized and contain pendant anionic carboxyl groups on the polymer backbone that enable the polymer to absorb aqueous fluids, such as body fluids.

In general, superabsorbent materials are manufactured by known polymerization techniques, preferably by polymerization in aqueous solution by gel polymerization. The products of this polymerization process are aqueous polymer gels (i.e., superabsorbent hydrogels) that are reduced in size to small particles by mechanical forces, then dried using drying procedures and apparatus known in the art. The drying process is followed by pulverization of the resulting superabsorbent material to the desired particle size.

To improve the fluid absorption profile, superabsorbent materials may be optimized with respect to one or more of absorption capacity, absorption rate, acquisition time, gel strength, and/or permeability. Optimization allows for a reduction in the amount of fluff fiber used in an absorbent article, which results in a thinner article. However, it is very difficult to maximize all of these absorption profile properties simultaneously.

One method of optimizing the fluid absorption profile of superabsorbent materials is to provide materials of a predetermined particle size distribution. In particular, particles that are too small in size swell after absorbing a fluid and can block the absorption of further fluid. Particles that are too large in size have a reduced surface area which decreases the rate of absorption. Therefore, the particle size distribution of the superabsorbent materials may be such that fluid permeability, absorption, and retention by the superabsorbent materials are maximized. Any subsequent process that agglomerates the superabsorbent materials to provide oversized particles should be avoided. In particular, agglomeration of superabsorbent materials increases apparent particle size, which reduces the surface area of the superabsorbent materials, and in turn adversely affects absorption of an aqueous fluid by the superabsorbent materials.

The article 20 can optionally further include a surge management layer (not shown) which may be located adjacent the absorbent core 44 and attached to various components in the article 20 such as the absorbent core 44 or the topsheet 42 by methods known in the art, such as by using an adhesive. In general, a surge management layer helps to quickly acquire and diffuse surges or gushes of liquid that may be rapidly introduced into the absorbent structure of the article. The surge management layer can temporarily store the liquid prior to releasing it into the storage or retention portions of the absorbent core 44. Examples of suitable surge management layers are described in U.S. Patents 5,486,166 to Bishop et al.; 5,490,846 to Ellis et al.; and 5,820,973 to Dodge et al.,

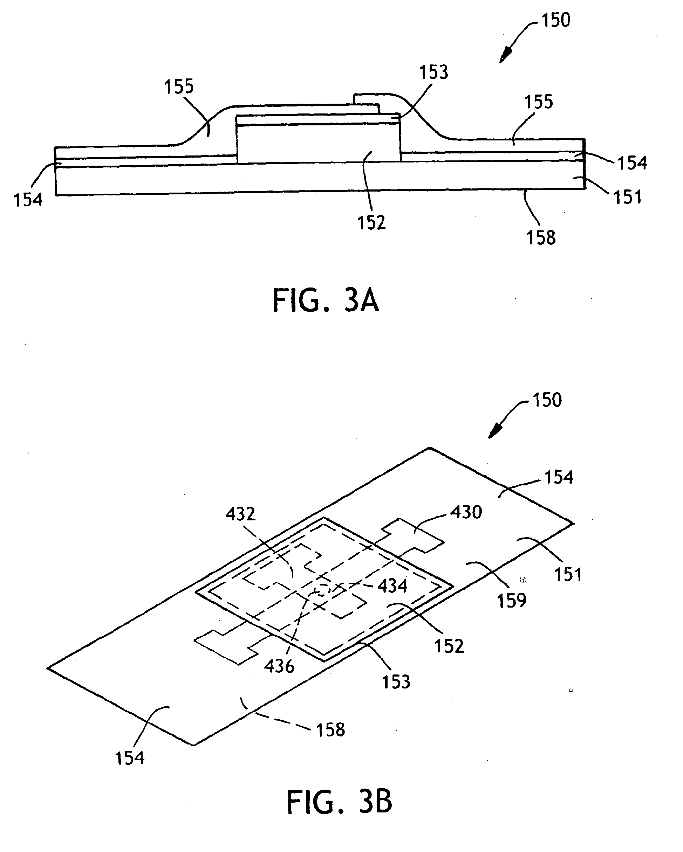

In addition to the disposable personal care article described above, another example of a disposable article may be a health/medical article such as a bandage. By way of a non-limiting example of the present invention, such a bandage, in some aspects, could comprise the invention of the present disclosure to provide feedback if excess bleeding occurs. Attention is directed to FIGs 3A and 3B, which show a possible configuration for a bandage. FIG 3A shows a cross-section view of the absorbent bandage with optional layers described below. FIG 3B shows a perspective view of the bandage with some of the optional or removable layers not being shown. The bandage 150 has a strip 151 of material having a body-facing side 159 and a second side 158 which is opposite the body-facing side. The strip is essentially a backsheet and is desirably prepared from the same materials described above for the backsheet. In addition, the strip may be an apertured material, such as an apertured film, or material which is otherwise gas permeable, such as a gas permeable film. The strip 151 supports the absorbent core 152 which is attached to the bodyfacing side 159 of the strip. In addition, an optional absorbent protective layer 153 may be applied to the absorbent core 152 and can be coextensive with the strip 151.

The bandage 150 may also have a pressuresensitive adhesive 154 applied to the body-facing side 159 of the strip 151. Any pressuresensitive adhesive may be used, provided that the pressuresensitive adhesive does not irritate the skin of the user. Suitably, the pressuresensitive adhesive is a conventional pressuresensitive adhesive which is currently used on similar conventional bandages. This pressuresensitive adhesive is preferably not placed on the absorbent core 152 or on the protective layer 153 in the area of the absorbent core 152. If the absorbent protective layer is coextensive with the strip 151, then the adhesive may be applied to areas of the protective layer 153 where the absorbent core 152 is not located. By having the pressuresensitive adhesive on the strip 151, the bandage is allowed to be secured to the skin of a user in need of the bandage. To protect the pressure sensitive adhesive and the absorbent, a release strip 155 can be placed on the bodyfacing side 159 of the bandage. The release liner may be removably secured to the article attachment adhesive and serves to prevent premature contamination of the adhesive before the absorbent article is secured to, for example, the skin. The release liner may be placed on the bodyfacing side of the bandage in a single piece (not shown) or in multiple pieces, as is shown in FIG 3A.

In another aspect of the present invention, the absorbent core of the bandage may be placed between a folded strip. If this method is used to form the bandage, the strip is suitably fluid permeable.

The bandage desirably also has a first circuit-path 430 disposed along a body-side surface of at least one substrate, and at least a second circuit-path 432 that is proximally adjacent a portion of the first circuit-path 430, and is disposed along an outward, garment-side. The circuit-paths are conductively connected by a hole 434 that has been filled with a conductive filler 436.

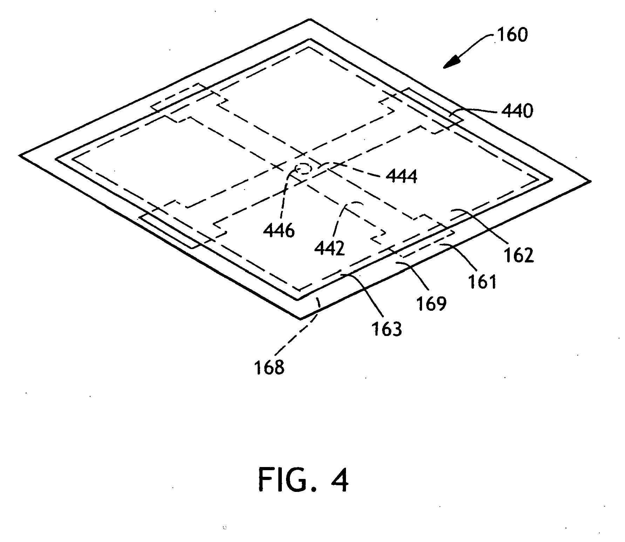

Houshold/industrial articles, such as absorbent furniture and/or bed pads or liners are also included within the present invention. By way of a non-limiting example of the present invention, such a pad or liner, in some aspects, could indicate when an incontinence insult is occurring. As is shown in FIG 4, a furniture or bed pad or liner 160 (hereinafter referred to as a "pad") is shown in perspective. The pad 160 has a fluid impermeable backsheet 161 having a furniture-facing side or surface 168 and an upwardfacing side or surface 169 which is opposite the furniture-facing side or surface 168. The fluid impermeable backsheet 161 supports the absorbent core 162 which is attached to the upward facing side 169 of the fluid impermeable backsheet. In addition, an optional absorbent protective layer 163 may be applied to the absorbent core. The optional substrate layer of the absorbent core can be the fluid impermeable layer 161 or the absorbent protective layer 163 of the pad. The pad desirably has a first circuit-path 440 disposed along a body-side surface of at least one substrate, and at least a second circuit-path 442 that is proximally adjacent a portion of the first circuit-path 440, and is disposed along an opposing side. The circuit-paths are conductively connected by a hole 444 that has been filled with a conductive filler 446.

To hold the pad in place, the furniture-facing side 168 of the pad may contain a pressuresensitive adhesive, a highfriction coating or other suitable material which will aid in keeping the pad in place during use. The pad can be used in a wide variety of applications including placement on chairs, sofas, beds, car seats and the like, to absorb any fluid which may come into contact with the pad.

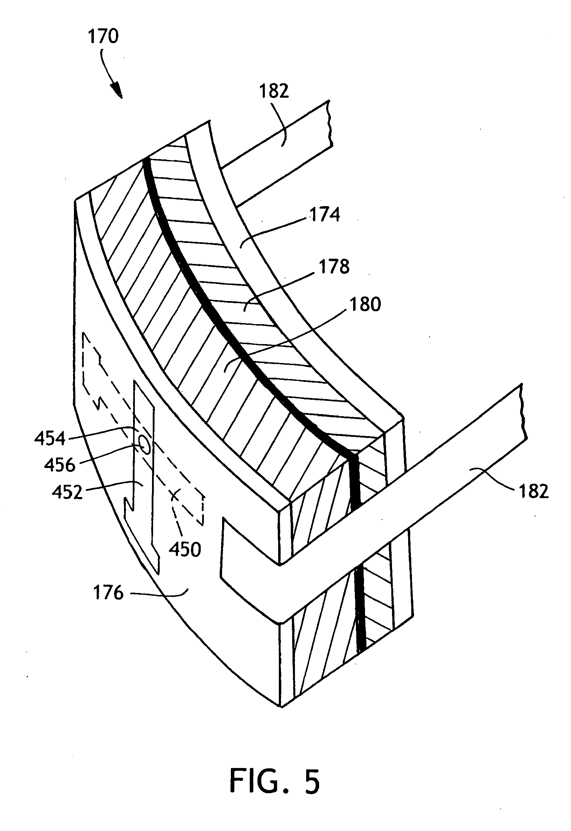

Sports/construction articles, such as an absorbent headband for absorbing perspiration or drying off equipment are also included within the present invention. By way of a non-limiting example of the present invention, the headband, in some aspects, could sense body temperature and sweat, and could predict the onset of heat exhaustion or heat stroke. As is shown in FIG 5, a sweatband 170 is shown in perspective. The sweatband 170 has an absorbent core 180 disposed between an optional topsheet 174 and/or an optional fluid impervious backsheet 176. In some aspects, the sweatband can have an additional layer 178, such as a fluid distribution layer, if desired. The optionally elastomeric nature of the article 170 allows the band to be fitted on the user's head or wrist while the nature of the invention retains exceptional dimensional stability to ensure contact with the skin. The low capacity region 178 can be positioned towards the user's skin and can maintain a comfortable feel to the user. VELCRO or other fastening device 182 can be used to facilitate adjustment or comfort. The sweatband desirably has a first circuit-path 450 disposed along a body-side surface of at least one substrate, and at least a second circuit-path 452 that is proximally adjacent a portion of the first disposed along an outward, garment-side. The circuit-paths are conductively connected by a hole 454 that has been filled with a conductive filler 456.

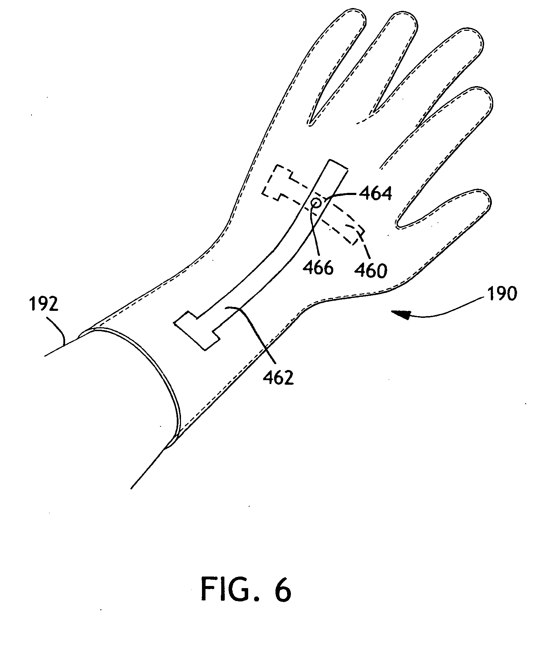

Disposable gloves, such as elastomeric gloves, are also included within the present invention. By way of a non-limiting example of the present invention, such a glove, in some aspects, could be capable of indicating the presence of static electricity. As shown in FIG 6, for example, one embodiment of an elastomeric glove 190 is illustrated that may be placed on the hand of a user 192. The glove 190 includes a substrate body 194 having the basic shape of the glove. The substrate body 194 may generally be formed from any of a variety of natural and/or synthetic elastomeric materials, known in the art. For instance, some examples of suitable elastomeric materials include, but are not limited to, S-EB-S (styrene-ethylene-butylene-styrene) block copolymers. S-I-S (styrene-isoprene-styrene) block copolymers, S-B-S (styrene-butadiene-styrene) block copolymers, S-1 (styrene- isoprene) block copolymers, S-B (styrene-butadiene) block copolymers, natural rubber latex, nitrile rubbers, isoprene rubbers, chloroprene rubbers, polyvinyl chlorides, silicone rubbers, and combinations thereof. Other suitable elastomeric materials that may be used to form the substrate body 194 may be described in U.S. Pat. No. 5,112,900 to Buddenhagen, et al.; U.S. Pat. No. 5, 407.7 1 5 to Buddenhagen, et al.; U.S. Pat. No. 5, 900,452 to Plamthottam; U.S. Pat. No. 6,288,159 to Plamthottam; and U.S. Pat. No. 6,306,514 to Weikel, et al., The glove desirably has a first circuit-path 460 disposed along a bodyside surface of the substrate, and at least a second circuit-path 462 that is proximally adjacent a portion of the first circuit-path 460, and is disposed along an outward-facing side. The circuit-paths are conductively connected by a hole 464 that has been filled with a conductive filler 466.

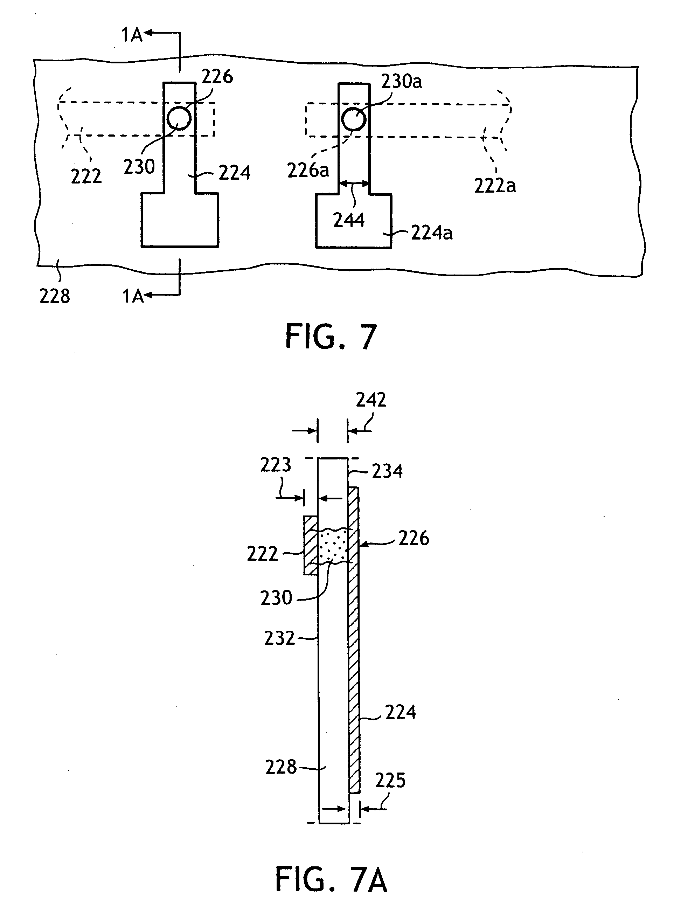

With reference to FIGs 7 through 12, a distinctive article and method for forming an operative, electrically-conductive path through the thickness of a substrate configured as an electrical barrier layer can comprise providing a first, electrically-conductive circuit-path 222; and separately providing a second, electrically-conductive circuit-path 224. A portion of the first circuit-path can be positioned proximally adjacent a portion of the second circuit-path at a first predetermined hole location 226, and a first, electrically-insulating barrier layer 228 can be operatively provided in a configuration which is interposed between the first circuit-path and the second circuit-path at the first hole location. The first circuit-path 222 has been conductively connected to the second circuit-path 224 at the first hole location 226, and the hole has been filled with a conductive filler to provide a first electrically conductive filler-path 230 between the first circuit-path 222 and the second circuit-path 224 through a thickness dimension 242 of at least the barrier layer 228 at the first hole location 226.

By incorporating its various aspects and features, alone or in desired combinations, the present invention can provide a desired, electrically-conductive interconnecting pathway between electrically-conductive circuit-paths that are positioned on opposite sides of an electrically-insulating barrier substrate. The invention can efficiently and economically interconnect selected circuit-paths through the thickness dimension of an intervening barrier layer of electrically-insulating material. The interconnecting-conductive pathway provided by the conductive filler can penetrate and extend through the thickness dimension of the substrate via the hole. In addition, the formation of the interconnecting-conductive pathway can be configured to operatively maintain desired properties of the substrate. For example, the formation of the interconnecting-conductive pathway can be configured to operatively maintain a desired liquid-impermeable barrier property of the substrate. As a result, the interconnecting-conductive pathway can be positioned at a greater range of locations and can help provide greater versatility. For example, when the conductive pathway interconnects a circuit-path that is positioned on one side of the insulating substrate to a cooperating sensor or other external electrical monitoring device that is positioned on an opposite side of the substrate, the conductive pathway can have a location that allows a convenient cooperative positioning of the monitoring device at a location that provides improved comfort to the wearer.

The first electrically-conductive circuit-path 222 can include a first electrically-conductive material, and the second electrically-conductive circuit-path 224 can include a second electrically-conductive material. The first electrically-conductive material and the second electrically-conductive material can be different or substantially the same, as desired. Similarly, any additional electrically conductive circuit-paths can include a corresponding electrically-conductive material, and each electrically-conductive material can be different or substantially the same as any other employed electrically-conductive material. In the various arrangements of the method, an individual circuit-path can include any operative conductive material. Suitable conductive materials can, for example, include gold, silver, copper, aluminum, nickel, cobalt, carbon-doped materials, conductive polymers or the like, as well as combinations thereof. The conductive materials may have the form of conductive foils, conductive laminates, conductive traces, conductive inks and the like, as well as combinations thereof.

It should be appreciated that an individual circuit-path may include additional components, which may be electronic or non-electronic. The electronic components may include passive components, such as resistors, capacitors, inductors or the like, as well as combinations thereof. The active components may include transistors, diodes, operational amplifiers, integrated-circuit components, microprocessor components and the like, as well as combinations thereof.

The first substrate configured to be an electrically-insulating barrier layer 228, as well as any other employed electrically-insulating layer, can be provided by employing any operative technique. For example, the selected barrier layer can be integrally formed with a corresponding conductive material employed in a corresponding circuit-path. Alternatively, the selected barrier layer can be a separately provided layer of barrier material that is subsequently assembled to a corresponding or associated circuit-path material. The barrier layer may be substantially monolithic or may include subcomponents, sub-layers, lamina, strata and the like, as desired.

In the various arrangements of the invention, an individual substrate that is configured to be an electrically-insulating barrier layer can include any operative, electrically-insulating material. Suitable electrically-insulating materials can include, for example, glass, glass fibers, cellulose, cellulose fibers, rubber, natural or synthetic elastomers, elastomeric fibers, plastics, polymer films and the like, as well as combinations thereof. In various aspects of the invention, any one or more layers of the disposable articles, such as those described above, can be configured to function as an electrically-insulating barrier layer.

With reference to FIGs 7 and 7A, the first electrically-conductive circuit-path 222 can be applied to a first substrate which is substantially nonconductive to electrical currents; and the first substrate can be configured to provide the first, electrically-insulating barrier layer 228. In a particular configuration, the first electrically-conductive circuit-path 222 can be applied to a first major, facing-surface 232 of the first substrate; and the second electrically-conductive circuit-path 224 can be applied to an opposite, second major facing-surface 234 of the first substrate.

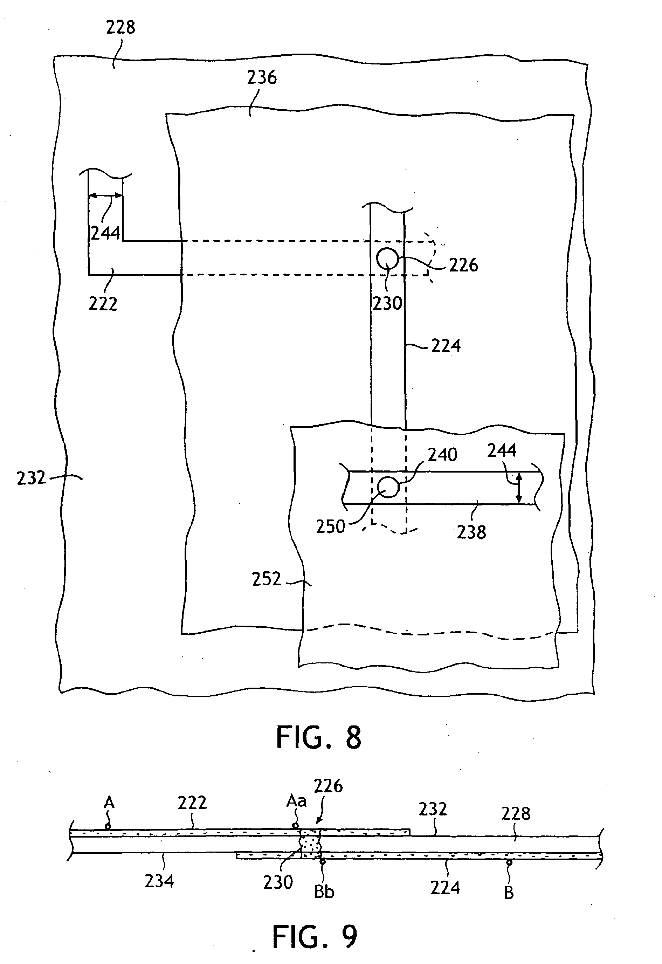

With further reference to FIG 8, an alternative arrangement can have the first, electrically-conductive circuit-path 222 applied to a first major, facing-surface 232 of the first substrate; and can have the second, separately provided electrically-conductive circuit-path 224 applied to a second substrate. In a desired feature, an individual substrate may be configured to be substantially nonconductive to electrical currents. Accordingly, the first substrate can be configured to provide a first, electrically-insulating barrier layer 228, and the second substrate can be configured to provide a second, electrically-insulating barrier layer 236.

Additionally, the invention may include providing a third electrically-conductive circuit-path 238, and positioning a portion of the second circuit-path 224 proximally adjacent a portion of the third circuit-path 238 at a second predetermined hole location 240. The second electrically-insulating barrier layer 236 can be interposed between the second circuit-path 224 and the third circuit-path 238 at the second hole location 240, and the second circuit-path 224 can be conductively connected to the third circuit-path 238 at the second hole location 240, with the conductive filler configured to operatively provide a second electrically-conductive filler-path 250 between the second circuit-path 224 and the third circuit-path 238 through the thickness dimension 242 (FIG 7A) of the second barrier layer 236 at the position of the second hole location 240.

In another aspect, with further reference to FIG 8, a representative configuration can have the first circuit-path 222 located on and operatively attached to a first substrate (e.g. barrier layer 228); a second circuit-path 224 located on and operatively attached to a second substrate (e.g. barrier layer 236); and a third circuit-path 238 located on and operatively attached to a third substrate (e.g. barrier layer 252). In particular aspects, the first circuit-path 222 can be operatively connected to the second circuit-path 224 along a first electrically-conductive conductive filler-path 230 which extends through the thickness of the second substrate 236. Additionally, the second circuit-path 224 can be operatively connected to the third circuit-path 238 along another separately provided electrically-conductive filler-path 250 which extends through the thickness of the third substrate 252. As a result, more complex arrays of circuit-paths can be more efficiently produced with fewer layers of substrate material. The reduced layers of substrate material can help reduce the thickness of the overall end-product, and can help increase the flexibility and comfort of the disposable article.

In a similar manner, additional electrically-conductive circuit-paths and additional electrically-insulating barrier layers may be stacked in alternating fashion to interpose the electrically-insulating barrier layers between corresponding circuit-paths at corresponding predetermined hole locations to provide further combinations and more complex configurations of the present invention. Portions of corresponding circuit-paths can be operatively positioned proximally adjacent each other at corresponding, predetermined hole locations, and a conductive filler, each of which may be substantially the same or different, can be employed in each hole to provide electrically conductive filler-paths between the circuit-paths.

An individual substrate layer or electrically-insulating barrier layer can be provided by any suitable material. For example, the substrate or barrier material can be any of the layers found in disposable articles of the present invention, such as those described above, and can include, for example, a polymer film, a woven fabric, a nonwoven fabric, a spunbond fabric, a meltblown fabric, a coform fabric material, an elastomeric film, an elastomeric composite material, a non-conductive coating, a non-conductive laminate or the like, as well as combinations thereof.

An individual substrate layer or barrier layer can include natural and/or synthetic materials. In desired arrangements, an individual substrate layer or barrier layer can include a synthetic polymer material. Such polymer materials can, for example, include polyethylene, polypropylene, polyester, melt-extrudable polymer fabrics or the like, as well as combinations thereof.

One feature of the article and method of the present invention can include configuring the individual substrate layer or electrically-insulating barrier layer with a selected flexibility value. The flexibility pertains to the ability to be flexed or bowed repeatedly without rupturing, and to an ease of bending, which can range from pliable (high flexibility) to stiff (low flexibility). The flexibility and "hand" of a material can relate to the tactile qualities of a fabric or other material. Such tactile qualities can, for example, include parameters of softness, firmness, elasticity, fineness, resilience as well as other qualities that are perceived by touch. For example, see the Dictionary of Fiber & Textile Technology, published by Hoechst Celanese Corporation, North Carolina in 1990.

To measure a flexure rigidity (or stiffness) of a material and to obtain a corresponding flexibility value, the employed test usually is typically referred to as a flex or bending test. In general, the flexure rigidity is a resistance to bending, or more specifically, the bending couple at either end of a strip of unit width that is bent into a unit curvature in the absence of any tension (ASTM D1388).

To determine the flexibility value of a selected substrate, the test material is flexed by a four-point loading, bending test (ref: Handbook of Polymer Testing, Brown, R; Marcel Dekker, Inc, New York, Basel, 1999). A suitable four-point loading, bending test is the KES Pure Bend test. This test can be conducted with a KAWABATA, model KES-FB-2 Bending Tester, or a substantially equivalent device. The KAWABATA Bending Tester is available from KATO TECH CO., LTD., a business having offices located in Kyoto, Japan, and the KAWABATA device includes detailed instructions for conducting the KES Pure Bend test.

The KES Pure Bend test has a high sensitivity to measure flexible materials. Since the KES test is a pure bending test, there are only normal stresses with no shearing stresses. In the KES pure bend test, the bending rigidity values are obtained from the initial slopes of the bending curve which is bending between curvatures of -2.5 cm$^{-1}$ and 2.5 cm$^{-1}$, with a constant rate of curvature change at 0.5 cm$^{-1}$/sec.

The bending rigidity can be employed to provide flexibility values, where:

$$\text{Bending Rigidity} = \text{averaged slope of the bending curve between}$$

$$\text{curvatures of } 0.5 \text{ cm}^{-1} \text{ and } 1.5 \text{ cm}^{-1}, \text{ and}$$

$$\text{curvatures of } -0.5 \text{ cm}^{-1} \text{ and } -1.5 \text{ cm}^{-1}.$$

In a particular aspect, the flexibility value (bending rigidity value) can be at least a minimum of about 0.0015 grams-force (gf) • cm$^2$/cm. In other aspects, the flexibility value can be up to a maximum of about 0.03 gf • cm$^2$/cm. The flexibility value can alternatively be up to about 0.028 gf • cm$^2$/cm, and can optionally be up to about 0.025 gf • cm$^2$/cm to provide desired effectiveness. If the flexibility value is outside the desired values, the material may have an excessively harsh feel. Additionally, the material may allow excessive fatigue stresses and strains on associated circuit-paths, circuit components or circuit connections.

An individual substrate layer or electrically-insulating barrier layer can be provided by a material having a selected softening point. For the purposes of the present disclosure, the softening point is the temperature at which the material can plastically flow in an operative manner. In a particular aspect, the softening point temperature of the individual substrate or barrier material can be at least a minimum of about 38°C. The softening point can alternatively be at least about 50°C, and can optionally be at least about 60°C to provide desired benefits. In other aspects, the softening point of the individual substrate or barrier material can be up to about 150°C, or more, to provide desired effectiveness.

A suitable determination of the softening point temperature of a material is the Vicat softening temperature, which can be measured in accordance with *ASTM D1525 - 06, Standard Test Method for Vicat Softening Temperature of Plastics.* Generally stated, the Vicat softening temperature is the temperature at which a flat-ended needle penetrates the specimen to the depth of 1 mm under a specific load. The temperature reflects the point of softening to be expected when a material is used in an elevated temperature application. During the testing, a test specimen is placed in the testing apparatus so that the penetrating needle rests on its surface at least 1 mm from the edge. A load of 10N or 50N is applied to the specimen. The specimen is then lowered into an oil bath at 23°C. The bath is raised at a rate of 50 °C per hour until the needle penetrates 1 mm. The test specimen is between 3 and 6.5 mm thick and at least 10 mm in width and length, and no more than three layers may be stacked to achieve a minimum thickness. The Vicat softening test determines the temperature at which the needle penetrates 1 mm. A suitable device for determining the softening point temperature can be an ATLAS HDV2 DTUL/Vicat tester (available from Plastics Technology Laboratories, Inc.

having a place of business in Pittsfield, Massachusetts, U.S.A.), or an equivalent device.

Referring to FIGs 7, 7A and 8, in some aspects of the invention, an individual electrically-conductive circuit-path, such as the first circuit-path 222 and/or the second circuit-path 224, can be applied by printing a corresponding electrically-conductive material from a liquid-state of the associated electrically-conductive material. For example, the first electrically-conductive circuit-path can be applied by printing a first electrically-conductive material from a liquid-state of the first electrically-conductive material, and the second electrically-conductive circuit-path can be applied by printing a second electrically-conductive material from a liquid-state of the second electrically-conductive material.

In some aspects, an individual, electrically-conductive material may include an electrically-conductive ink. The conductive ink includes electrically-conductive materials and can be formulated for printing onto the selected substrate using various printing processes. The conductive ink typically includes a vehicle including one or more resins and/or solvents. Various other ink additives known in the art, e.g., antioxidants, leveling agents, flow agents and drying agents, may be included in the conductive ink. The conductive ink can be in the form of a paste, slurry or dispersion. The ink generally also include; one or more solvents that readily can be adjusted by the skilled practitioner for a desired rheology. The ink formulation is desirably mixed in a grinding mill to sufficiently wet the conductive particles with the vehicle, e.g., solvent and resin.

The conductive material can include silver, copper, gold, palladium, platinum, carbon and the like, or combinations of these particles. The average particle size of the conductive material can be within the range of between about 0.5 $\mu$m and about 20 $\mu$m, such as between about 2 $\mu$m and about 5 $\mu$m, or about 3 $\mu$m. The amount of conductive material in the conductive trace or circuit-path can be between about 60% and about 90%, such as between about 75% and about 85%, on a dry weight basis.

The electrically-conductive particles can be flakes and/or powders. In particular arrangements, the conductive flakes have a mean aspect ratio of between about 2 and about 50. such as between about 5 and about 15. The aspect ratio is a ratio of the largest linear dimension of a particle to the smallest linear dimension of the particle. For example, the aspect ratio of an ellipsoidal particle is the diameter along its major axis divided by the diameter along its minor axis. For a flake, the aspect ratio is the longest dimension across the length of the flake divided by its thickness.

Suitable conductive flakes may include those sold by METALOR (a business having offices located in Attleboro, Massachusetts, U.S.A.) under the following trade designation: P185-2 flakes having a particle size distribution substantially between about 2 $\mu$m and about 18 $\mu$m; P264-1 and P264-2 flakes having particle size distributions substantially between about 0.5 $\mu$m and about 5 $\mu$m; P204-2 flakes having a particle size distribution substantially between about 1 $\mu$m and about 10 $\mu$m; P204-3 flakes having a particle size distribution substantially between about 1 $\mu$m and about 8 $\mu$m; P204-4 flakes having a particle size distribution substantially between about 2 $\mu$m and about 9 $\mu$m; EA-2388 flakes having a particle size distribution substantially between about 1 $\mu$m and about 9 $\mu$m; SA-0201 flakes having a particle size distribution substantially between about 0.5 $\mu$m and about 22 $\mu$m and having a mean value of about 2.8 $\mu$m; RA-0001 flakes having a particle size distribution substantially between about 1 $\mu$m and about 6 $\mu$m; RA-0015 flakes having a particle size distribution substantially between about 2 $\mu$m and about 17 $\mu$m; and RA-0076 flakes having a particle size distribution substantially between about 2 $\mu$m and about 62 $\mu$m, and having a mean value of about 12 $\mu$m.

Suitable silver powders may include those sold by METALOR under the following trade designations: C-0083P powder having a particle size distribution substantially between about 0.4 $\mu$m and about 4 $\mu$m, and having a mean value of about 1.2 $\mu$m; K-0082P powder having a particle size distribution substantially between about 0.4 $\mu$m and about 6.5 $\mu$m, and having a mean value of about 1.7 $\mu$m; and K-1321P powder having a particle size distribution substantially between about 1 $\mu$m and about 4 $\mu$m.

The conductive ink may include a resin. Suitable resins can include, for example, polymers, polymer blends, fatty acids or the like, as well as combinations thereof. In particular arrangements, alkyd resins may be employed. Examples of such resins include LV-2190, LV-2183 and XV-1578 alkyd resins from Lawter International (a business having offices located in Kenosha, Wisconsin, U.S.A.). Also suitable are Crystal Gloss Metallic Amber resin, Z-kyd resin, and alkali refined linseed oil resin available from Kerley Ink (a business having offices located in Broadview, Illinois, U.S.A.). Soy resins, such as those available from Ron Ink Company (a business having offices located in Rochester, New York, U.S.A.) are also suitable.

Solvents for use in the conductive ink formulation are well known in the art, and a person can readily identify a number of suitable solvents for use in a particular printing application. Solvents can generally comprise between about 3% and about 40% of the ink by weight on a wet basis. The amount may vary depending on various factors including, but not limited to, the viscosity of the resin, the solvation characteristics of the solvent, and the conductive particle size, distribution and surface morphology for any given printing method. Generally, the solvent can be added to the ink mixture until a desired ink rheology is achieved. The desired rheologies can depend on the printing method used, and are well known by skilled printers and ink manufacturers.

The solvent in the conductive ink can include non-polar solvents such as a hydrocarbon solvent, water, an alcohol such as isopropyl alcohol, and combinations thereof. Particular arrangements may employ an aliphatic hydrocarbon solvent. Examples of suitable solvents include ISOPAR H aliphatic hydrocarbon solvent from Exxon Corporation (a business having offices located in Houston, Texas, U.S.A.); EXX-PRINT M71a and EXX-PRINT 274a aliphatic and aromatic hydrocarbon solvent from Exxon Corporation; and MCGEE SOL 52, MCGEE SOL 47 and MCGEE SOL 470 aliphatic and aromatic hydrocarbon solvent from Lawter International (having a place of business in Kenosha, Wisconsin, U.S.A.).

Various printing techniques can be employed to produce an individual, electrically- conductive circuit-path or trace. The printing techniques are conventional and commercially available. For example, the electrically-conductive ink can be applied to the selected substrate using printing techniques known in the art for printing inks on paper and other substrates, including, but not limited to, offset-lithographic (wet, waterless and dry), flexographic, rotogravure (direct or offset), intaglio, ink jet, electrophotographic (laser jet and photocopy), bead, spray, vapor deposition, screen printing, printing plate contact, letterpress printing and the like. These printing methods are desirable because conventional methods for forming traces on circuit boards include multiple steps (e.g., photoresist, cure and etching) are time intensive, environmentally unfriendly, and relatively expensive: Commercial printing presses preferably are used for printing on the substrates of the present invention. Commercial printing presses may require additional drying capability to dry the ink after printing or require modifications to handle polymer films (e.g., to handle electrostatic charge). These types of modifications are known in the art and typically can be ordered when purchasing a commercial printing press. Depending on the printing technology, printing speed in the range of from about 150 feet per minute (45.7 meters per minute) to about 300 feet per minute (91.4 meters per minute) readily can be achieved. It is envisioned that even greater printing speeds can be achieved, e.g., about 1000 feet per minute (304.8 meters per minute) or more.

The conductive ink printing pattern can be registered to graphics and other product components. Temperature, electric charge, magnetisms or other means can be used to control the conductive ink printing placement. Wider print areas called landing zones can be printed in areas where the hole penetration through the film occurs to ensure proper coverage of printing.

The electrically-conductive ink can desirably be deposited in a quantity such that the dried conductive trace or circuit-path has a thickness dimension which is within the range of about 1 $\mu$m to about 8 $\mu$m, depending on the printing process used. For example, a single printing operation which provides an ink film thickness of about 2 $\mu$m to about 3 $\mu$m is typically sufficient to achieve adequate conductivity. The conductive ink optionally can be printed on the selected substrate two or more times to deliver more conductive ink to the selected substrate. In particular aspects, the conductive ink is printed only once to avoid the registration problems that may arise when printing multiple times.

Optionally, the conductive ink may be dried at a selected drying temperature to help form the desired conductive trace or circuit-path. In a particular aspect, the drying can be conducted prior to a step of embedding the trace into its associated, cooperating substrate. The drying temperature is desirably selected to avoid excessive damage to the substrate or barrier layer material. The conductive ink may be dried at the selected drying temperature to drive off some or all of the solvent or carrier to minimize any bubbles containing trapped solvent, and/or to minimize pin holes or craters from forming in the conductive circuit-path due to rapid solvent evaporation. The conductive ink can be dried using an oven, such as a convection oven, or using infrared, and radio frequency drying, or ultraviolet (UV) radiation. In a particular aspect, the heating device may be designed to allow the printed substrate to pass through so that the conductive ink can be dried in a continuous manner to facilitate large-scale production. The drying temperature employed depends on the ink used, the softening temperature of the selected substrate, and the drying time or belt speed. Typical drying temperatures can be within the range of about 125°F to about 150°F (about 52°C - 66°C). When UV is employed, the drying temperature may be at room temperature.

After the drying step, the circuit element can be allowed to cool prior to the optional embedding step. Alternatively, the drying step can be achieved continuously with the embedding step as the trace is heated to the drying temperature.

At least one hole located at a first predetermined hole location can be formed which extends through at least one substrate which is desirably configured to be an electrically-insulating barrier layer. In some aspects, a substrate of the invention may include multiple holes. In other aspects, the invention may include multiple substrates, which may further include multiple circuit-paths. In still other aspects, the invention may include multiple substrates having multiple holes and/or multiple circuit-paths. In yet other aspects, the invention may include multiple flexible substrates that are interposed between the first circuit-path and second circuit-path at the first hole location. It is desirable that each hole extends completely through the thickness of the substrate(s), such that a conductive connection can be made with each circuit-path. However, it is not necessary that the diameter of the hole remains constant therethrough.

The hole can be formed using means known to those skilled in the art. Such means can include, but is not limited to, aperturing, die cutting, ultrasonics, localized stretching, high pressure gas, high pressure liquid, electromagnetic particle excitation, radio frequency, and the like, or combinations thereof. In general, the hole needs to be large enough so that the conductive filler material fills the hole and provides an electrically-conductive path between the circuit-paths, but small enough to form a fluid-tight seal, if desired. The average diameter of the hole can vary based on numerous factors, including conductive filler material composition, substrate material composition, substrate flexibility, substrate softening temperature, substrate thickness, conductive filler resistance, the desired current that needs to be passed through the substrate, and the like. In some desired aspects, the hole diameter is about 0.1 mm or less, such as between about 1 Angstrom (Å) and about 0.1 mm to provide desired effectiveness. (Note that 1 Å = 1 x 10$^{-9}$ meter.)

The hole is desirably filled with a conductive filler that is capable of sufficiently conducting an electric current such that an adequate connection is achieved between the first circuit-path and the proximally adjacent second circuit-path. The desired allowable resistance within the conductive filler will vary based on the application. In addition, in some aspects, the conductive filler can also be capable of plugging the hole such that a liquid-impermeable substrate remains liquid-impermeable, if desired.

The conductive filler is generally a substance made of a conductive material. Suitable conductive materials include, but are not limited to, gold, silver, copper, palladium, platinum, aluminum, nickel, cobalt, carbon, carbon-doped materials, conductive polymers, and the like, or combinations thereof. The conductive filler, when applied to the hole, may be in the form of a solid or a liquid, and can include, but is not limited to, slurries, dispersions, gels, pastes and the like.

The conductive filler can be applied to the hole using suitable means known in the art. Such means include, but are not limited to, printing, rolling, extrusion, injection, spraying and the like, or combinations thereof. In some aspects, at least one of the circuit-paths, such as a printed circuit-path, could also function as the conductive filler, provided that it extends through the hole such that the first circuit-path is conductively connected to the second circuit-path.

Referring to FIGs 7, 7A and 8, in desired configurations of the invention, the individual conductive materials in at least the portions of the circuit-paths located at an individual hole location (226, 240), can have a selected thickness dimension. As representatively shown, for example, the conductive material of the first circuit-path 222 can have a thickness 223, and the conductive material of the second circuit-path 224 can have a thickness 225. In a particular aspect, the combined thickness of the conductive materials in the circuit-paths can be of at least a minimum of about 9 $\mu$m, such as at least about 10 $\mu$m, or at least about 12 $\mu$m to provide improved performance. In other aspects, the combined thickness of the conductive materials can be up to about 50 $\mu$m, or more, to provide desired benefits.

In some aspects, the conductive materials in at least portions of the circuit-paths can have a combined thickness which is a selected percentage of the thickness of the substrate or barrier layer that is interposed between the circuit-paths. In particular aspects, the combined thickness can be at least a minimum of about 5% of the thickness of the interposed substrate or barrier layer, such as at least about 2 % of the thickness of the interposed substrate or barrier layer, or at least about 50% of the thickness of the interposed substrate or barrier layer to provide improved performance. In other aspects, the combined thickness of the conductive materials can be up to about 60%, or more, of the thickness of the interposed substrate or barrier layer to provide desired benefits.

In still other aspects, an individual circuit-path (e.g. circuit-path 222, 224 and/or 238) can be provided with a selected cross-machine direction (CD) width dimension 244. In particular aspects, the CD width can be at least about 0.01 cm, such as at least about 0.02 cm, or at least about 0.03 cm. In other aspects, the CD width can be less than about 1 cm, such as less than about 0.5 cm or less than about 0.1 cm to provide desired effectiveness. If the CD width of an individual circuit-path is outside the desired values, the cost of the conductive material can be excessively high. As a result, the manufacturing costs of the end product can be outside the desired ranges. It should be readily appreciated that the areas and width dimensions of an individual circuit-path, can be determined by employing standard microscopy techniques which are well known in the art.

An individual, electrically-conductive circuit-path (e.g. circuit-path 222 and/or circuit-path 224) can have a selected electrical resistivity value, particularly in a generally proximal vicinity of its corresponding hole location. In a desired aspect, the resistivity value can be substantially zero $\Omega$/m (ohms/meter). In other aspects, the electrical resistivity value can be less than about 1 M$\Omega$/m (mega-ohms/meter), such as less than about 1 K$\Omega$/m (kilo-ohms/ meter), or less than about 100 $\Omega$/m to provide improved effectiveness.

In other aspects, the resistivity value of an individual, electrically-conductive circuit-path can be substantially zero ohms per square per mil of the electrically-conductive material ($\Omega$/square per mil). (Note that 1 mil = 0.001 inch.) In still other aspects, the resistivity value can be as low as 0.1 $\Omega$/square per mil, such as 1 $\Omega$/square per mil. In yet other aspects, the resistivity value can be less than about 33 K$\Omega$/square per mil, such as less than about 16 K$\Omega$/square per mil, or less than about 8 K$\Omega$/square per mil to provide desired effectiveness. A suitable procedure for determining the resistivity values in terms of "ohms per square per mil" is *ASTM F 1896 - 98* (*Reapproved 2004*), *Test Method for Determining the Electrical Resistivity of a Printed Conductive Material.*

Another aspect of the present invention can have a configuration in which an individual, electrically-conductive path (e.g., conductive filler-path 230 and/or 250) has been configured to provide a selected electrical resistance value. For example, the resistance value can be as low as zero ohms, such as at least about 0.1 $\Omega$, or at least about 0.5 $\Omega$ to provide desired performance. In other aspects, the electrical resistance value can be less than about 1 K$\Omega$, such as less than about 100 $\Omega$, or less than about 10 $\Omega$ to provide desired performance.

If the resistance value is too large, or is otherwise outside the desired values, excessive power consumption and excessive design complexity can arise. Additionally, the measurement sensitivity and measurement accuracy in associated circuits may be degraded.

With reference to FIG 9, the resistance of an individual conductive filler-path can be determined by employing the following *Resistance Measurement Test* procedure: (a) Measure the resistance of the first circuit-path from an operative point A, to a point Aa that is immediately adjacent the conductive filler-path and is relatively closest to point A, to determine a first, resistance value R1. (b) Measure the resistance of the second circuit-path from an operative point B, to a point Bb that is immediately adjacent the conductive filler-path and is relatively closest to point B, to determine a second, resistance value R2. (c) Measure the overall resistance along a path that runs directly from point A to Aa, directly through the conductive filler-path, and directly from point Bb to point B, to determine a total resistance value $R_T$,

Then: $R_B = R_T - R1 - R2$; where $R_B$ = resistance of the conductive filler-path that is interposed between point A and point B.

The hole location can have any operative configuration. For example, in some aspects, the conductive filler in the hole location can have any operative shape. The shape of the conductive filler therethrough may be irregular or substantially regular.

In some aspects, the hole filled with conductive filler can be configured to be substantially liquid-impermeable. More particularly, the hole filled with conductive filler can be substantially liquid-impermeable in a region which extends over the conductive filler area and past the perimeter of the hole by a distance of about 1 mm. In some aspects, an operatively liquid-impermeable conductive filler can have a formulation and construction which is capable of supporting a hydrohead of at least about 45 cm of water without allowing a significant level of leakage. A suitable technique for determining the resistance of a material to liquid penetration is *Federal Test Method Standard FTMS 191 Method 5514.* dated 31 December 1968, or a substantially equivalent procedure.

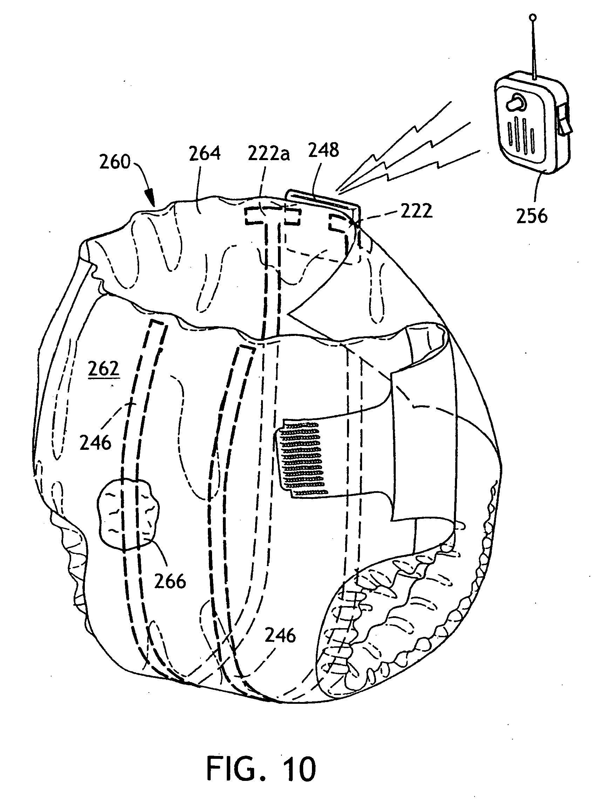

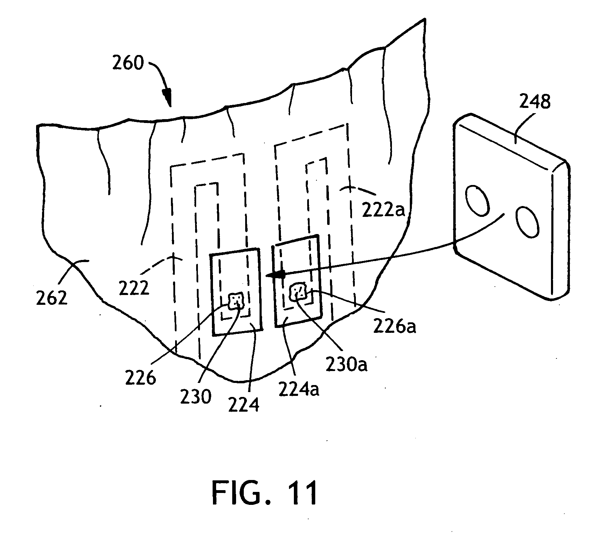

With reference to FIG 10 and FIG 11, some aspects of the present invention can include at least one electrically-conductive circuit-path (e.g. circuit-path 222) which has been operatively connected to a sensor mechanism 246 which can provide selected sensor data. In other aspects, at least another electrically conductive circuit-path (e.g. circuit-path 224) can be operatively connected to an electronic processor mechanism 248 which can operatively receive the sensor data and provide selected signal data.

Any appropriate detecting, sensing or interrogating device or system may be operatively employed to provide the sensor mechanism 246 that is incorporated with the present invention. A suitable sensor mechanism can, for example, include a wetness sensor, a motion sensor, a temperature sensor, a humidity sensor, a pressure sensor, a position sensor, a proximity sensor, a light sensor, an odor sensor or the like, as well as combinations thereof.

It is understood that any appropriate information or data may be operatively included in the sensor data that is generated with the invention. Suitable sensor data can, for example, include data regarding resistance, voltage, capacitance, inductance, wetness, motion, temperature, humidity, pressure, position, proximity, light, odor and the like, as well as combinations thereof. Any appropriate analyzing, computing or assessing device or system may also be operatively included with the electronic processor mechanism 248. A suitable electronic processor mechanism can, for example, include a micro controller, a micro processor, an analog to digital converter, a Field Programmable Gate Array (FPGA), an Electrically Erasable Programmable Read-Only Memory (EEPROM), an electronic memory device and the like, as well as combinations thereof. The electronic processor can collect, process, store, analyze and convert digital or analog data, provide feedback and the like, as well as combinations thereof.

It is understood that any desired information or data may be included in the signal data that is generated. Suitable signal data can include, for example, data pertaining to light, sound, tactile, odor, electrical impulses, biometric data, motion, vibration, wireless communication and the like, as well as combinations thereof. In some aspects, the electronic processor mechanism 248 may be configured to transfer the signal data to another, relatively remote location. As representatively shown, for example, the invention may be configured to transmit signal data with a wireless communication link to a remote receiver device 256.

FIG 10 and FIG 11 illustrate an example of a suitable article, such as the representatively shown personal care article, which can be configured to incorporate the present invention. As representatively shown, the article 260 can, for example, be configured to provide an infant diaper or a child-care training pant. The article can have an outercover 262, a first circuit-path disposed along a body-side surface of the outercover, and at least a second circuit-path disposed along an outward, garment-side surface of the outercover. As representatively shown, a first supplemental circuit-path 222a can be disposed along the bodyside surface of the outercover; and a second supplemental circuit-path 224a can be disposed along the outward, garment-side surface of the outercover.

In some aspects, the first circuit-paths 222 and 222a can be operatively connected to a selected sensor mechanism. In this particular aspect, the sensor mechanism could be a wetness sensor. The sensor mechanism can, for example, be configured to provide one or more functions or operations pertaining to a wireless, audio, visual and/or tactile indication of a monitored event. Additionally, the sensor mechanism can, for example, be configured to provide one or more functions or operations pertaining to a number of events, lengths of times between events, as well as any other statistics pertaining to a selected event, as desired by a user. As representatively shown, the sensor mechanism can be an internal sensor that is configured to detect a presence of aqueous liquid, which is within the article 260 and is present above a selected threshold level.

Additionally, the second circuit-paths 224 and 224a can be operatively connected to a selected electronic processor mechanism. In the representatively shown arrangement, for example, the electronic processor mechanism can be a microcontroller. The electronic processor mechanism can, for example, be configured to convert data (Analog to Digital, or Digital to Analog), store data, trigger a predetermined response, allow for user interrupt, provide signal conditioning, compute and process algorithms and the like, as well as combinations thereof.

As representatively shown, at least a selected portion of the first circuit-path (222 and/or 222a) is positioned proximally adjacent at least an operative portion of the second circuit-path (224 and/or 224a) at a first predetermined hole location (226 and/or 226a). The backsheet 262 has a position that is interposed between the first and second circuit-paths, and is composed of a material that provides an electrically-insulating barrier layer which is interposed between the first circuit-path and second circuit-path at the first hole location 226 and/or 226a. The first circuit-paths 222 and/or 222a are configured to operatively connect to the second circuit-paths 224 and/or 224a through the thickness dimension of the outercover 262 with a conductive filler positioned therein the first hole location 226 and/or 226a. The conductive filler is configured to provide an electrically-conductive interconnecting path 230 and/or 230a between the appointed first circuit-path and the appointed second circuit-path at the first hole location 226 and/or 226a. As representatively shown, a separately provided external electronic processor mechanism 248 can be operatively connected to the second circuit-paths 224 and/or 224a. In some aspects, the electronic processor mechanism 248 can be removably attached or otherwise removably connected to the second circuit-paths 224 and/or 224a on the outside surface of the outercover 262. Accordingly, the electrically-conductive interconnecting path can be employed to operatively connect the internally positioned sensor mechanism to the separately provided, external, electronic processor mechanism with an operative, electrically-conductive connection.

The article 260 can also include a topsheet or bodyside liner layer 264, and an absorbent core 266 positioned between the outercover layer 262 and topsheet layer 264. Additionally, the article 260 can include other components, such as fasteners, elastic members, transfer layers, distribution layers or the like, as desired, in conventional arrangements that are well known in the art.

**EXAMPLES**

The following examples are provided to illustrate the invention, and do not limit the scope of the claims.

Example 1

A first copper based circuit-path having a length of approximately 10 cm, a width of approximately 10 mm, and a thickness in the range of approximately 0.1 $\mu$m to about 0.4 $\mu$m was printed by ink jet printing onto one side of a flexible substrate. The substrate which functions as a backsheet for current HUGGIES PULL-UPS STEP 3 training pants was approximately 0.75 mils (19 $\mu$m) thick and had a flexibility in the range of approximately 0.0017 to 0.0021 gf • cm$^2$/cm as measured by the KES Pure Bend test (described above). A hole measuring approximately 0.1 mm in diameter was formed in the the substrate at a location in the middle of the length of the first circuit-path (approximately 5 cm from each end) using a stick pin. A second circuit-path of PERMATEX Quick Grid Rear Window Defogger Repair Kit (available from Permatex, Inc., having a place of business in Hartford, Connecticut, U.S.A.) having a length of approximately 10 cm, a width of approximately 6 mm, and a thickness in the range of approximately 5 $\mu$m to about 50 $\mu$m was then applied to the opposite side of the substrate using the brush supplied with the PERMATEX Quick Grid Rear Window Defogger Repair Kit. The second circuit-path was located such that the hole was in the middle of the length of the second circuit-path, and the second circuit-path was proximally adjacent a portion of the first circuit-path. Each circuit path intersected the other at the location of the hole. An amount of the PERMATEX was also forced into and through the hole such that it completely filled the hole and was in contact with the first circuit-path to function as a conductive filler for the hole, thus providing a continuous electrical circuit between the first and second circuit-paths. The PERMATEX was then allowed to air-dry until it became solid.

Using a FLUKE Model # 189 True RMS Multimeter, the resistance of the first circuit-path, the second circuit-path, the total resistance of the entire interconnected circuit, and the conductive filler path were determined using the Resistance Measurement Test procedure described above. The first circuit-path exhibited a resistance in the range of 5-10 ohms, the second circuit-path exhibited a resistance in the range of 5-10 ohms, the total resistance of the entire interconnected circuit was in the range of 15-30 ohms, and the conductive filler-path was determined to be between 5-10 ohms. This example demonstrates, among other things, that a continuous circuit had in fact been established, and that the resistance of the components was consistent.

Example 2

A circuit made according to Example 1 was draped over a standard lab beaker having a circular top opening approximately 70 mm wide and approximately 200 mm tall such that the substrate formed a hemisphere with the hole containing conductive filler at the bottom of the hemisphere and centered on the beaker. The substrate hemisphere was filled with water and allowed to sit for 10 minutes. The water was then drained off of the top of the film and the film was removed from the beaker. The beaker under the film was found to be bone dry. This Example demonstrates that the invention maintains the fluid impervious properties of the substrate.

It will be appreciated that details of the foregoing examples, given for purposes of illustration, are not to be construed as limiting the scope of this invention. Although, only a few exemplary embodiments of this invention have been described in detail above, those skilled in the art will readily appreciate that many modifications are possible in the examples without materially departing from the novel teachings and advantages of this invention.

## Claims

1. An article selected from personal care absorbent articles, health/medical absorbent articles, household/industrial absorbent articles and sports/construction articles, said article comprising:

    a flexible substrate configured to provide a first electrically-insulating barrier layer;
    wherein the first electrically-insulating barrier layer has a first electrically-conductive circuit-path, a second electrically-conductive circuit-path and at least one hole which penetrates through the entire thickness of the first electrically-insulating barrier layer;
    wherein a portion of the second circuit-path is proximally adjacent a portion of the first circuit-path at a first predetermined hole location;
    wherein the flexible substrate is interposed between the first circuit-path and second circuit-path at the first hole location; and
    wherein the at least one hole is filled with a conductive filler to form an interconnecting conductive filler-path between the first circuit-path and the second circuit-path such that the first circuit-path is conductively connected to the second circuit-path at the first predetermined hole location to form a continuous electrical circuit.

2. The article of claim 1 wherein multiple flexible substrates are interposed between the first circuit-path and second circuit-path at the first hole location.

3. The article of claim 1 wherein the flexible substrate has a flexibility value of about 0.0015 gf $\cdot$ cm$^2$/cm to about 0.03 gf $\cdot$ cm$^2$/cm.

4. The article of claim 1 wherein the combined thickness of the conductive materials at the first hole location is between about 9$\mu$m to about 50 $\mu$m.

5. The article of claim 1 wherein the combined thickness of the conductive materials at the first hole location is about 5% to about 60% of the thickness of the interposed substrate.

6. The article of claim 1 wherein the first circuit path is connected to a sensor.

7. The article of claim 1 wherein the second circuit-path is connected to an electronic processor mechanism which receives sensor data and provides selected signal data:

8. The article of any of the preceding claims wherein said article is a disposable article comprising:

    A topsheet, a backsheet and an absorbent core disposed between and in facing relation to the topsheet and the backsheet;
    wherein at least one of the topsheet, backsheet and absorbent core is said flexible substrate.

9. The disposable article of claim 8 wherein the absorbent core comprises between about 30% and about 90% by weight of superabsorbent material.

10. A method, comprising:

    providing a first electrically-conductive circuit-path;
    separately providing a second electrically-conductive circuit-path;
    positioning a portion of the first circuit-path proximally adjacent a portion of the second circuit-path at a first predetermined hole location;

providing a first electrically-insulating barrier layer interposed between the first circuit-path and second circuit-path at the first hole location;

providing a hole which penetrates through the entire thickness of the first electrically-insulating barrier layer at the first hole location;

filling the hole with a conductive filler to conductively connect the first circuit-path to the second circuit-path at the first hole location to provide an electrically-conductive filler-path between the first circuit-path and the second circuit-path at the first hole location.

11. The method of claim 10 wherein

the first electrically-conductive circuit-path has been applied to a first substrate which is substantially nonconductive to electrical currents; and

the first substrate has been configured to provide the first, electrically-insulating barrier layer.

12. The method of claim 10 wherein the first substrate has been provided by a first film or nonwoven fabric material having a softening point temperature of up to about 150°C and a flexibility value of up to about 0.03 gf • cm$^2$/cm.

13. The method of claim 10 wherein the second separately provided electrically-conductive circuit-path has been applied to a second substrate which is substantially nonconductive to electrical currents.

14. The method of claim 13 wherein the second substrate has been provided by a first film or nonwoven fabric material having a flexibility value of up to about 0.03 gf • cm$^2$/cm.

15. The method of claim 10 wherein

the first electrically-conductive circuit-path has been operatively connected to a sensor mechanism which provides sensor data; and

the second electrically-conductive circuit-path has been operatively connected to an electronic processor mechanism which receives the sensor data and provides signal data.

16. The method of claim 10 wherein the first hole location has been configured to be substantially liquid-impermeable.

17. The method of claim 10 wherein

the first electrically-conductive circuit-path has been applied to a first substrate which is substantially nonconductive to electrical currents;

the first substrate has been configured to provide the first, electrically-insulating barrier layer;

the first electrically-conductive circuit-path has been applied to the first substrate by printing a first electrically-conductive material from a liquid-state of the first electrically-conductive material;

the first substrate has been provided by a first substrate material having a flexibility value of up to about 0.03 gf • cm$^2$/cm.

the conductive filler has been provided to conductively connect the first circuit-path to the second circuit-path at the first hole location;

the first electrically-conductive circuit-path has a resistivity of not more than about 100 Ω/m;

the first filler-path has a resistance value of not more than about 1 KΩ between the first circuit-path and second circuit-path; and

the first hole location is substantially liquid-impermeable.

**Patentansprüche**

1. Ein Artikel ausgewählt aus absorptionsfähigen Körperpflegeartikeln, absorptionsfähigen Gesundheits-/Medizinartikeln, absorptionsfähigen Haushalts/- Industrieartikeln und Sport-/Konstruktionsartikel, wobei der Artikel umfasst:

ein flexibles Substrat, welches eingerichtet ist, um eine erste elektrisch isolierende Sperrschicht bereitzustellen, wobei die erste elektrisch isolierende Sperrschicht einen ersten elektrisch leitenden Schaltkreispfad, einen zweiten elektrisch leitenden Schaltkreispfad und mindestens ein Loch aufweist, welches die gesamte Dicke der ersten elektrisch isolierenden Sperrschicht durchdringt, wobei ein Teil des zweiten elektrisch leitenden Schaltkreispfades nahe angrenzend an einen Teil des ersten Schaltkreispfades an einer ersten vorbestimmten Lochposition ist;

wobei das flexible Substrat zwischen dem ersten Schaltkreispfad und dem zweiten Schaltkreispfad an der ersten Lochposition eingefügt ist; und

wobei das mindestens eine Loch mit einem leitenden Füllmittel gefüllt ist, um einen verbindenden leitenden Füllmittelpfad zwischen dem ersten Schaltkreispfad und dem zweiten Schaltkreispfad zu bilden, so dass der erste Schaltkreispfad mit dem zweiten Schaltkreispfad an der ersten vorbestimmten Lochposition leitend verbunden ist, um einen kontinuierlichen elektrischen Schaltkreis zu bilden.

2. Der Artikel gemäß Anspruch 1, wobei mehrere flexible Substrate zwischen den ersten Schaltkreispfad und den zweiten Schaltkreispfad an der ersten Lochposition eingefügt sind.

3. Der Artikel gemäß Anspruch 1, wobei das flexible Substrat einen Flexibilitätswert von ungefähr 0,0015 gf·cm$^2$/cm bis ungefähr 0,03 gf·cm$^2$/cm aufweist.

4. Der Artikel gemäß Anspruch 1, wobei die kombinierte Dicke der leitfähigen Materialien an der ersten Lochposition zwischen ungefähr 9 $\mu$m bis ungefähr 50 $\mu$m beträgt.

5. Der Artikel gemäß Anspruch 1, wobei die kombinierte Dicke der leitfähigen Materialien an der ersten Lochposition ungefähr 5% bis ungefähr 60% der Dicke der eingefügten Substrate ist.

6. Der Artikel gemäß Anspruch 1, wobei der erste Schaltkreispfad mit einem Sensor verbunden ist.

7. Der Artikel gemäß Anspruch 1, wobei der zweite Schaltkreispfad mit einem elektronischen Prozessormechanismus verbunden ist, der Sensordaten empfängt und ausgewählte Datensignale bereitstellt.

8. Der Artikel gemäß einem der vorherigen Ansprüche, wobei der Artikel ein Einwegartikel ist, welcher umfasst:

eine obere Bahn, eine hintere Bahn und einen absorptionsfähigen Kern, der zwischen und in zugewandter Beziehung zu der oberen Bahn und der hinteren Bahn angeordnet ist;

wobei mindestens eines von der oberen Bahn, der hinteren Bahn oder dem absorptionsfähigen Kern das flexible Substrat ist.

9. Der Einwegartikel gemäß Anspruch 8, wobei der absorptionsfähige Kern zwischen ungefähr 30 Gew.-% und ungefähr 90 Gew.-% superabsorptionsfähiges Material umfasst.

10. Ein Verfahren, welches umfasst:

Bereitstellen eines ersten elektrisch leitenden Schaltkreispfades;

separates Bereitstellen eines zweiten elektrisch leitenden Schaltkreispfades;

Positionieren eines Teils des ersten Schaltkreispfades nahe angrenzend an einem Teil des zweiten Schaltkreispfades an einer ersten vorbestimmten Lochposition;

Bereitstellen einer ersten elektrisch isolierenden Sperrschicht, welche zwischen dem ersten Schaltkreispfad und dem zweiten Schaltkreispfad an der ersten Lochposition eingefügt ist;

Bereitstellen eines Lochs, welches die gesamte Dicke der ersten elektrisch isolierenden Sperrschicht an der ersten Lochposition durchdringt;

Füllen des Lochs mit einem leitfähigen Füllmaterial, um den ersten Schaltkreispfad mit dem zweiten Schaltkreispfad an der ersten Lochposition leitend zu verbinden, um einen elektrisch leitenden Füllmittelpfad zwischen dem ersten Schaltkreispfad und dem zweiten Schaltkreispfad an der ersten Lochposition zu bilden.

11. Das Verfahren gemäß Anspruch 10, wobei

der erste elektrisch leitende Schaltkreispfad auf ein erstes Substrat aufgebracht wurde, welches für elektrische Ströme im Wesentlichen nicht leitend ist, und

wobei das erste Substrat eingerichtet wurde, um die erste elektrisch isolierende Sperrschicht bereitzustellen.

12. Das Verfahren gemäß Anspruch 10, wobei das erste Substrat durch einen ersten Film oder Vliesstoffmaterial bereitgestellt wurde, welcher/welches eine Erweichungspunkttemperatur von bis zu ungefähr 150°C und einen Flexibilitätswert von bis zu ungefähr 0,03 gf·cm$^2$/cm aufweist.

13. Das Verfahren gemäß Anspruch 10, wobei der zweite, separat bereitgestellte elektrisch leitende Schaltkreispfad auf ein zweites Substrat aufgebracht wurde, welches im Wesentlichen für elektrische Ströme nicht leitend ist.

**14.** Das Verfahren gemäß Anspruch 13, wobei das zweite Substrat durch einen ersten Film oder Vliesstoffmaterial bereitgestellt wurde, welcher/welches einen Flexibilitätswert von bis zu ungefähr 0,03 gf·cm$^2$/cm aufweist.

**15.** Das Verfahren gemäß Anspruch 10, wobei

der erste elektrisch leitende Schaltkreispfad mit einem Sensormechanismus wirkverbunden wurde, welcher Sensordaten bereitstellt; und

wobei der zweite elektrisch leitende Schaltkreispfad mit einem elektronischen Prozessormechanismus wirkverbunden wurde, welcher die Sensordaten empfängt und Signaldaten bereitstellt.

**16.** Das Verfahren gemäß Anspruch 10, wobei die erste Lochposition eingerichtet wurde, um im Wesentlichen flüssigkeitsundurchlässig zu sein.

**17.** Das Verfahren gemäß Anspruch 10, wobei

der erste elektrisch leitende Schaltkreispfad auf ein erstes Substrat aufgebracht wurde, welches für elektrische Ströme im Wesentlichen nicht leitend ist,

das erste Substrat eingerichtet wurde, die erste elektrisch isolierende Sperrschicht bereitzustellen;

der erste elektrisch leitende Schaltkreispfad auf das erste Substrat aufgetragen wurde durch Drucken eines ersten elektrisch leitenden Materials von einem flüssigen Zustand des ersten elektrisch leitenden Materials;

das erste Substrat durch ein erstes Substratmaterial bereitgestellt wurde, welches einen Flexibilitätswert von bis zu ungefähr 0,03 gf·cm$^2$/cm aufweist;

das leitende Füllmaterial bereitgestellt wurde, um den ersten Schaltkreispfad mit dem zweiten Schaltkreispfad an der ersten Lochposition leitend zu verbinden;

der erste elektrisch leitende Schaltkreispfad einen Widerstand von nicht mehr als ungefähr 100Ω/m aufweist;

der erste Füllmaterialpfad einen Widerstandswert von nicht mehr als 1 KΩ zwischen dem ersten Schaltkreispfad und dem zweiten Schaltkreispfad aufweist; und

die erste Lochposition im Wesentlichen flüssigkeitsundurchlässig ist.

## Revendications

**1.** Article sélectionné parmi les articles absorbants d'hygiène personnelle, les articles absorbants sanitaires/médicaux, les articles absorbants domestiques/industriels et les articles de sport/de construction, ledit article comprenant :

un substrat souple configuré pour fournir une première couche formant barrière isolante de l'électricité ; la première couche formant barrière isolante de l'électricité ayant un premier tracé de circuit conducteur de l'électricité, un second tracé de circuit conducteur de l'électricité et au moins un trou qui pénètre au travers de l'entière épaisseur de la première couche formant barrière isolante de l'électricité ; une portion du second tracé de circuit étant adjacente, de façon proximale, à une portion du premier tracé de circuit au niveau d'un premier emplacement prédéterminé de trou ;

le substrat souple étant interposé entre le premier tracé de circuit et le second tracé de circuit au niveau du premier emplacement de trou ;

ledit au moins un trou étant rempli d'une charge conductrice pour former un tracé de charge conducteur d'interconnexion entre le premier tracé de circuit et le second tracé de circuit de telle sorte que le premier tracé de circuit est connecté, de manière conductrice, au second tracé de circuit au niveau du premier emplacement prédéterminé de trou pour former un circuit électrique continu.

**2.** Article selon la revendication 1, dans lequel de multiples substrats souples sont interposés entre le premier tracé de circuit et le second tracé de circuit au niveau du premier emplacement de trou.

**3.** Article selon la revendication 1, dans lequel le substrat souple a une valeur de souplesse comprise entre environ 0,0015 gf•cm$^2$/cm et environ 0,03 gf•cm$^2$/cm.

**4.** Article selon la revendication 1, dans lequel l'épaisseur combinée des matériaux conducteurs au niveau du premier emplacement de trou est comprise entre environ 9 $\mu$m et environ 50 $\mu$m.

**5.** Article selon la revendication 1, dans lequel l'épaisseur combinée des matériaux conducteurs au niveau du premier emplacement de trou est comprise entre environ 5 % et environ 60 % de l'épaisseur du substrat interposé.

**6.** Article selon la revendication 1, dans lequel le premier tracé de circuit est connecté à un capteur.

**7.** Article selon la revendication 1, dans lequel le second tracé de circuit est connecté à un mécanisme à processeur électronique qui reçoit des données de capteur et fournit des données de signaux sélectionnés.

**8.** Article selon l'une quelconque des revendications précédentes, ledit article étant un article jetable comprenant :

une feuille supérieure, une feuille dossier et un noyau absorbant disposé entre la feuille supérieure et la feuille dossier et en vis-à-vis de celles-ci ;
l'un au moins de la feuille supérieure, de la feuille dossier et du noyau absorbant est constitué dudit substrat souple.

**9.** Article jetable selon la revendication 8, dans lequel le noyau absorbant comprend entre environ 30 % et environ 90 % en poids de matériau superabsorbant.

**10.** Procédé comprenant :

la fourniture d'un premier tracé de circuit conducteur de l'électricité ;
la fourniture séparée d'un second tracé de circuit conducteur de l'électricité ;
le positionnement d'une portion du premier tracé de circuit adjacente, de façon proximale, à une portion du second tracé de circuit au niveau d'un premier emplacement prédéterminé de trou ;
la fourniture d'une première couche formant barrière isolante de l'électricité interposée entre le premier tracé de circuit et le second tracé de circuit au niveau du premier emplacement de trou ;
la prévision d'un trou qui pénètre au travers de l'entière épaisseur de la première couche formant barrière isolante de l'électricité au niveau du premier emplacement de trou ;
le remplissage du trou avec une charge conductrice qui connecte, de manière conductrice, le premier tracé de circuit au second tracé de circuit au niveau du premier emplacement de trou pour réaliser un tracé de charge conductrice de l'électricité entre le premier tracé de circuit et le second tracé de circuit au niveau du premier emplacement de trou.

**11.** Procédé selon la revendication 10, dans lequel
le premier tracé de circuit conducteur de l'électricité a été appliqué à un premier substrat qui est sensiblement non conducteur des courants électriques ; et
le premier substrat a été configuré pour réaliser la première couche formant barrière isolante de l'électricité.

**12.** Procédé selon la revendication 10, dans lequel le premier substrat a été réalisé au moyen d'un premier matériau en film ou en étoffe non-tissée ayant une température de point de ramollissement allant jusqu'à environ 150°C et une valeur de souplesse allant jusqu'à environ 0,03 gf•cm$^2$/cm.

**13.** Procédé selon la revendication 10, dans lequel le second tracé de circuit conducteur de l'électricité, fourni séparément, a été appliqué à un second substrat qui est sensiblement non conducteur des courants électriques.

**14.** Procédé selon la revendication 13, dans lequel le second substrat a été réalisé au moyen d'un matériau en film ou étoffe non-tissée ayant une valeur de souplesse allant jusqu'à environ 0,03 gf•cm$^2$/cm.

**15.** Procédé selon la revendication 10, dans lequel
le premier tracé de circuit conducteur de l'électricité a été connecté opérationnellement à un mécanisme capteur qui fournit des données de capteur ; et
le second tracé de circuit conducteur de l'électricité a été connecté opérationnellement à un mécanisme à processeur électronique qui reçoit les données de capteurs et fournit des données de signaux.

**16.** Procédé selon la revendication 10, dans lequel le premier emplacement de trou a été configuré pour être sensiblement imperméable aux liquides.

**17.** Procédé selon la revendication 10, dans lequel
le premier tracé de circuit conducteur de l'électricité a été appliqué à un premier substrat qui est sensiblement non conducteur des courants électriques :

le premier substrat a été configuré pour fournir la première couche formant barrière isolante de l'électricité ;
le premier tracé de circuit conducteur de l'électricité a été appliqué au premier substrat en imprimant un premier matériau conducteur de l'électricité à partir d'un état liquide du premier matériau conducteur de l'électricité ;
le premier substrat a été pourvu d'un premier matériau substrat ayant une valeur de souplesse allant jusqu'à environ 0,03 gf•cm$^2$/cm ;
la charge conductrice a été réalisée de façon à connecter, de manière conductrice, le premier tracé de circuit au second tracé de circuit au niveau du premier emplacement de trou ;
le premier tracé de circuit conducteur de l'électricité a une résistivité qui n'excède pas environ 100 Ω/m ;
le premier tracé de charge a une valeur de résistance qui n'excède pas environ 1KΩ entre le premier tracé de circuit et le second tracé de circuit ; et
le premier emplacement de trou est sensiblement imperméable aux liquides.

# FIG. 1

FIG. 2

FIG. 3A

FIG. 3B

FIG. 4

FIG. 5

FIG. 6

FIG. 7

FIG. 7A

228

236

244

222

232

230

226

224

240 244

250

238

252

## FIG. 8

A 222 Aa 226 232 228

234 230 Bb 224 B

## FIG. 9

FIG. 10

FIG. 11

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5883028 A, Morman
- US 5116662 A, Morman
- US 5114781 A, Morman
- US 6552245 B, Roessler
- US 6641134 B, Vukos
- US 3849241 A, Butin
- US 5350624 A, Georger
- US 4100324 A, Anderson
- US 4587154 A, Hotchkiss
- US 4604313 A, McFarland
- US 4655757 A, McFarland
- US 4724114 A, McFarland
- GB 2151272 A, Minto
- US 6362389 B, D. J. McDowall
- US 883174 A, X. Zhang
- US 5486166 A, Bishop
- US 5490846 A, Ellis
- US 5820973 A, Dodge
- US 5112900 A, Buddenhagen
- US 5407715 A, Buddenhagen
- US 5900452 A, Plamthottam
- US 6288159 B, Plamthottam
- US 6306514 B, Weikel

**Non-patent literature cited in the description**

- Dictionary of Fiber & Textile Technology. Hoechst Celanese Corporation, 1990
- **Brown, R.** Handbook of Polymer Testing. Marcel Dekker, Inc, 1999